# EUROPEAN PATENT APPLICATION

(11) **EP 4 202 052 A1**
(43) Date of publication of application: **28.06.2023**
(21) Application number: 21860265.4
(22) Date of filing: 19.08.2021
(51) Int. Cl.: C12P 19/04, C12P 19/14, C12N 15/54

(54) **STARCH BIOSYNTHESIS METHOD**

(30) Priority: 24.08.2020 CN 202010858974
(71) Applicant: Tianjin Institute of Industrial Biotechnology, Chinese Academy of Sciences, Tianjin Airport Economic Area Tianjin 300308 (CN)
(72) Inventor: MA, Yanhe, Tianjin 300308 (CN); CAI, Tao, Tianjin 300308 (CN); SUN, Hongbing, Tianjin 300308 (CN); QIAO, Jing, Tianjin 300308 (CN); ZHANG, Fan, Tianjin 300308 (CN); ZHANG, Jie, Tianjin 300308 (CN); WANG, Qinhong, Tianjin 300308 (CN)
(74) Representative: Gille Hrabal Partnerschaftsgesellschaft mbB Patentanwälte
(86) International application number: PCT/CN2021/113598
(87) International publication number: WO 2022/042427

(57) **Abstract**

Provided is a starch biosynthesis method. The method may implement total artificial biosynthesis from simple compounds such as dihydroxyacetone, formaldehyde, formic acid and methanol to starch, and by coupling with methods such as chemical reduction of carbon dioxide, even total artificial biosynthesis of starch taking carbon dioxide as a starting raw material can be implemented. Natural starch synthesis is required to be subjected to the Calvin cycle, needing 21-22 reaction steps in total, and the present method merely needs 9-12 reaction steps. Nearly a half of the reaction steps is reduced, and a production cycle is greatly shortened. In addition, the present method can utilize carbon dioxide of high concentration and high density and electric energy and hydrogen energy of high energy density, is more suitable for an industrial production mode, and the production cycle is shortened from several months in farming to several days.

## Description

The present application claims priority to the prior Application with the patent application No. 202010858974.9 entitled "STARCH BIOSYNTHESIS METHOD" and filed with China National Intellectual Property Administration on Aug. 24, 2020, which is incorporated herein by reference in its entirety.

### TECHNICAL FIELD

The present disclosure belongs to the technical field of biosynthesis, and particularly relates to a biosynthesis method for starch (including amylose and amylopectin).

### BACKGROUND

In global food production, 38% of land and 70% of fresh water resources needs to be consumed. The global demand for food is expected to have increased with the world population by 50-70% by 2050. It is difficult to meet the rising demand for food with the current agricultural planting mode. Starch is an important ingredient of food, providing about half the energy required by the human body per day. In addition, starch is an important starting material for the biological manufacturing and production of chemicals, for example, for the production of amino acids, organic acids and bioethanol and the like.

Agricultural planting is currently the only way to produce starch. The natural way of synthesizing starch in crops including the Calvin cycle involves a total of more than twenty chemical reactions and metabolic intermediates as well as numerous organelles. Although there are reports saying the synthesis of starch is achieved, the starting materials such as glucose 1-phosphate, adenosine diphosphate glucose, sucrose and dextrin and the like are still obtained from agricultural planting, and therefore these reported methods are not capable of producing starch in place of agricultural planting. The development of methods capable of producing starch in place of agricultural planting is of great significance.

Traveling in space and exploring the universe are always dreams for human beings, but how to supply food under space conditions has always been a great challenge. Major space powers have been concerned with developing methods of supplying food based on plant cultivation. However, due to the constrains of the way crops produce starch, it requires dozens to hundreds of cubic meters of space to meet a single person's demand for starch. The development of new ways of synthesizing starch will help achieve food supply under space conditions.

As metabolic engineering and synthetic biology have developed, many drugs and agricultural products such as Artemisinin, opium, lycopene, milk and meat and the like can be produced by industrial fermentation. However, the industrial synthesis of starch has not been achieved.

Carbon dioxide is the major greenhouse gas and also an important carbon starting material. About 10 billion tons of carbon dioxide are emitted every year in China. It can be converted and utilized as an important industrial starting material. Efficient chemical catalysts have been developed in China and other countries. They can reduce carbon dioxide to simple compounds such as formic acid and methanol and the like by means of light, electricity and hydrogen energy. However, complex compounds such as starch cannot be further synthesized using chemical catalysts.

Therefore, there were needs in the art for a scientific way of utilizing one-carbon compounds to reduce the emission of carbon dioxide in one respect and an industrial way of achieving the biosynthesis of starch in another respect. At present, there has been no technique or method that can meet both of these needs.

### SUMMARY

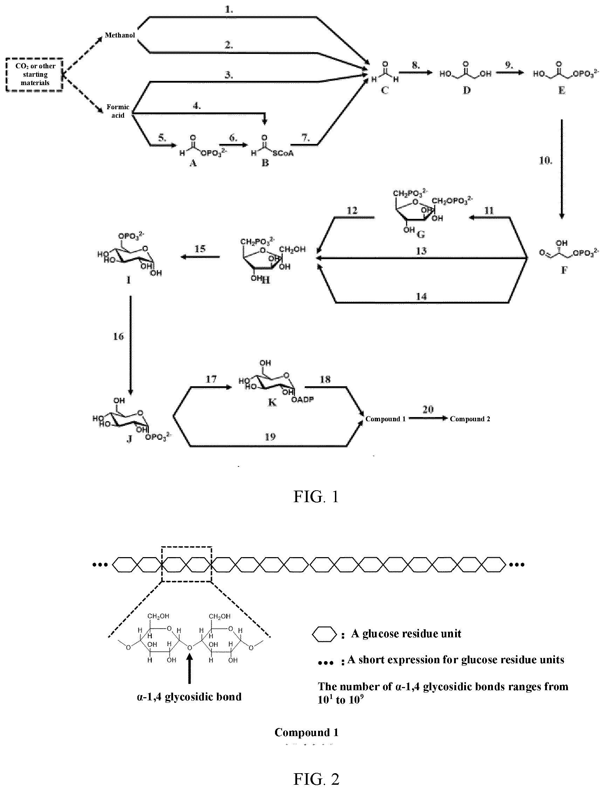

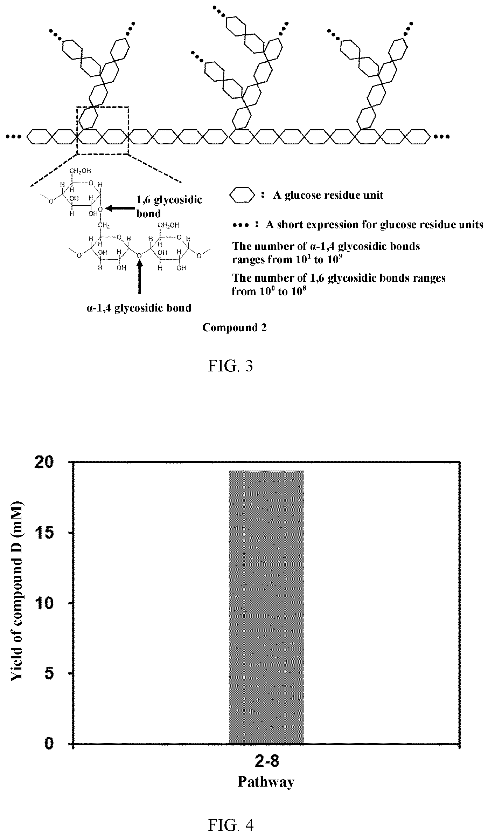

The present disclosure provides a new way for the synthesis of starch, which achieves the synthesis of amylose (FIG. 2) and/or amylopectin (FIG. 3) from a one-carbon compound through the combination of different chemical reactions.

The present disclosure provides a method (I) for the synthesis of starch, which comprises steps of converting a starting material compound D, namely dihydroxyacetone, into starch by catalysis with multiple enzymes;
the method specifically comprises the following steps:
step (1): converting the starting material compound D, namely dihydroxyacetone, into a compound F, namely D-glyceraldehyde 3-phosphate, by catalysis with one or more enzymes;
step (2): converting the compound F obtained in step (1) into a compound I, namely D-glucose-6-phosphate, by catalysis with one or more enzymes; and
step (3): converting the compound I obtained in step (2) into starch by catalysis with one or more enzymes.

According to an embodiment of the method (I) of the present disclosure, the enzyme used in step (1) is an enzyme or a combination of enzymes which catalyzes conversion of dihydroxyacetone into D-glyceraldehyde 3-phosphate by one-step or multi-step reaction. For example, the enzyme may be a combination of an enzyme having the function of catalyzing conversion of dihydroxyacetone into dihydroxyacetone phosphate and an enzyme having the function of catalyzing conversion of dihydroxyacetone phosphate into D-glyceraldehyde 3-phosphate.

According to an embodiment of the method (I) of the present disclosure, the enzyme used in step (2) is an enzyme or a combination of enzymes which catalyzes conversion of D-glyceraldehyde 3-phosphate into D-glucose-6-phosphate by one-step or multi-step reaction. For example, the enzyme may be the following enzyme combinations: an enzyme combination (I-2-a): a combination of an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate and an enzyme having the function of catalyzing conversion of D-fructose-6-phosphate into D-glucose-6-phosphate; or an enzyme combination (I-2-b): a combination of an enzyme having the function of catalyzing conversion of D-glyceraldehyde 3-phosphate into D-fructose-6-phosphate and an enzyme having the function of catalyzing conversion of D-fructose-6-phosphate into D-glucose-6-phosphate.

According to an embodiment of the method (I) of the present disclosure, the enzyme used in step (3) is an enzyme or a combination of enzymes which catalyzes conversion of D-glucose-6-phosphate into amylose or amylopectin by one-step or multi-step reaction. For example, the enzyme may be the following enzyme combinations:
an enzyme combination (I-3-a): a combination of an enzyme having the function of catalyzing conversion of D-glucose-6-phosphate into α-D-glucose-1-phosphate and an enzyme having the function of catalyzing conversion of α-D-glucose-1-phosphate into amylose; or
an enzyme combination (I-3-b): a combination of an enzyme having the function of catalyzing conversion of D-glucose-6-phosphate into α-D-glucose-1-phosphate, and enzyme having the function of catalyzing conversion of α-D-glucose-1-phosphate into adenosine diphosphate-α-D-glucose and an enzyme having the function of catalyzing conversion of adenosine diphosphate-α-D-glucose into amylose;
optionally, the combination (I-3-a) or (I-3-b) further comprises an enzyme having the function of catalyzing conversion of amylose into amylopectin.

According to an embodiment of the method (I) of the present disclosure, step (1) comprises the following sub-steps:
step (1-1): converting the compound D into a compound E, namely dihydroxyacetone phosphate, by catalysis with one or more enzymes (the reaction is denoted by reaction 9); and
step (1-2): converting the compound E obtained in step (1-1) into the compound F, namely D-glyceraldehyde 3-phosphate, by catalysis with one or more enzymes (the reaction is denoted by reaction 10).

According to an embodiment of the method (I) of the present disclosure, the enzyme used in step (1-1) is an enzyme having the function of catalyzing conversion of dihydroxyacetone into dihydroxyacetone phosphate.

According to an embodiment of the method (I) of the present disclosure, the enzyme used in step (1-2) is an enzyme having the function of catalyzing conversion of dihydroxyacetone phosphate into D-glyceraldehyde 3-phosphate.

According to an embodiment of the method (I) of the present disclosure, step (2) comprises the following sub-steps:
step (2-1): converting the compound F into a compound H, namely D-fructose-6-phosphate, by catalysis with one or more enzymes; and
step (2-2): converting the compound H obtained in step (2-1) into the compound I, namely D-glucose-6-phosphate, by catalysis with one or more enzymes (the reaction is denoted by reaction 15).

According to an embodiment of the method (I) of the present disclosure, the enzyme used in step (2-1) is an enzyme or a combination of enzymes which catalyzes conversion of D-glyceraldehyde 3-phosphate into D-fructose-6-phosphate by one-step or multi-step reaction. For example, the enzyme may be a single enzyme having the function of catalyzing conversion of D-glyceraldehyde 3-phosphate into D-fructose-6-phosphate, or may be an enzyme combination (I-2-1): a combination of an enzyme having the function of catalyzing conversion of D-glyceraldehyde 3-phosphate into D-fructose-1,6-bisphosphate and an enzyme having the function of catalyzing conversion of D-fructose-1,6-bisphosphate into D-fructose-6-phosphate.

Specifically, step (2-1) may be done by: converting the compound F into the compound H by catalysis with an enzyme having the function of catalyzing conversion of D-glyceraldehyde 3-phosphate into D-fructose-6-phosphate (the reaction is denoted by reaction 13 or 14). Reactions 13 and 14 are catalyzed by different enzymes; for example, the reaction of converting D-glyceraldehyde 3-phosphate into D-fructose-6-phosphate by catalysis with fructose 6-phosphate aldolase (FSA) or transaldolase is denoted by reaction 13, and the reaction of converting D-glyceraldehyde 3-phosphate into D-fructose-6-phosphate by catalysis with fructose 6-phosphate aldolase phosphatase (FBAP) is denoted by reaction 14.

Alternatively, step (2-1) may also be done by: first, converting the compound F into a compound G, namely D-fructose-1,6-bisphosphate, by catalysis with an enzyme having the function of catalyzing conversion of D-glyceraldehyde 3-phosphate into D-fructose-1,6-bisphosphate (the reaction is denoted by reaction 11), and then converting the compound G into D-fructose-6-phosphate by catalysis with an enzyme having the function of catalyzing conversion of D-fructose-1,6-bisphosphate into D-fructose-6-phosphate (the reaction is denoted by reaction 12). According to an embodiment of the method (I) of the present disclosure, the enzyme used in step (2-2) is an enzyme having the function of catalyzing conversion of D-fructose-6-phosphate into D-glucose-6-phosphate.

According to an embodiment of the method (I) of the present disclosure, step (3) comprises the following sub-steps:
step (3-1): converting the compound I into a compound J, namely α-D-glucose-1-phosphate, by catalysis with one or more enzymes (the reaction is denoted by reaction 16);
step (3-2): converting the compound J obtained in step (3-1) into a compound 1, namely amylose, by catalysis with one or more enzymes; and
optional step (3-3): converting the compound 1 obtained in step (3-2) into a compound 2, namely amylopectin, by catalysis with one or more enzymes (the reaction is denoted by reaction 20). According to an embodiment of the method (I) of the present disclosure, the enzyme used in step (3-1) is an enzyme having the function of catalyzing conversion of D-glucose-6-phosphate into α-D-glucose-1-phosphate.

According to an embodiment of the method (I) of the present disclosure, the enzyme used in step (3-2) is an enzyme having the function of catalyzing conversion of α-D-glucose-1-phosphate into amylose. For example, the enzyme may be a single enzyme having the function of catalyzing conversion of α-D-glucose-1-phosphate into amylose, or may be an enzyme combination (I-3-2): a combination of an enzyme having the function of catalyzing conversion of α-D-glucose-1-phosphate into adenosine diphosphate-α-D-glucose and an enzyme having the function of catalyzing conversion of adenosine diphosphate-α-D-glucose into amylose.

Specifically, step (3-2) may be done by: converting the compound J into the compound 1, namely amylose, by catalysis with an enzyme having the function of catalyzing conversion of α-D-glucose-1-phosphate into amylose (the reaction is denoted by reaction 19). Alternatively, step (3-2) may also be done by: first, converting the compound J into a compound K, namely adenosine diphosphate-α-D-glucose, by catalysis with an enzyme having the function of catalyzing conversion of α-D-glucose-1-phosphate into adenosine diphosphate-α-D-glucose (the reaction is denoted by reaction 17), and then converting the obtained compound K into amylose by catalysis with an enzyme having the function of catalyzing adenosine diphosphate-α-D-glucose into amylose (the reaction is denoted by reaction 18).

According to an embodiment of the method (I) of the present disclosure, the enzyme used in step (3-3) is an enzyme having the function of catalyzing conversion of amylose into amylopectin. According to an embodiment of the method (I) of the present disclosure, the method of the present disclosure further comprises, before step (1), step (0) of converting starting material formaldehyde into the compound D, namely dihydroxyacetone, by catalysis with one or more enzymes (the reaction is denoted by reaction 8).

According to an embodiment of the method (I) of the present disclosure, the enzyme used in step (0) is an enzyme having the function of catalyzing conversion of formaldehyde into dihydroxyacetone.

According to an embodiment of the method (I) of the present disclosure, the method of the present disclosure further comprises, before step (0), step (a) of converting starting material methanol or formic acid into formaldehyde by catalysis with one or more enzymes.

According to an embodiment of the method (I) of the present disclosure, in step (a), when methanol is used as a starting material to synthesize formaldehyde, the enzyme used is an enzyme having the function of catalyzing conversion of methanol into formaldehyde.

According to an embodiment of the method (I) of the present disclosure, in step (a), when formic acid is used as a starting material to synthesize formaldehyde, the enzyme used is an enzyme having the function of catalyzing conversion of formic acid into formaldehyde. For example, the enzyme may be a single enzyme having the function of catalyzing conversion of formic acid into formaldehyde, or may be an enzyme combination (I-a-1): a combination of an enzyme having the function of catalyzing conversion of formic acid into formyl coenzyme A and an enzyme having the function of catalyzing conversion of formyl coenzyme A into formaldehyde, or an enzyme combination (I-a-2): a combination of an enzyme having the function of catalyzing conversion of formic acid into formyl phosphate and an enzyme having the function of catalyzing conversion of formyl phosphate into formaldehyde.

Specifically, in step (a), when methanol is used as a starting material to synthesize formaldehyde, the reaction can be catalyzed by alcohol oxidase or alcohol dehydrogenase. The reaction catalyzed by alcohol dehydrogenase is denoted by reaction 1, and the reaction catalyzed by alcohol oxidase is denoted by reaction 2.

Specifically, in step (a), when formic acid is used as a starting material to synthesize formaldehyde, any one of the following steps (a1), (a2) and (a3) can be conducted:
step (a1): converting starting material formic acid into formaldehyde by catalysis with an enzyme having the function of catalyzing conversion of formic acid into formaldehyde (the reaction is denoted by reaction 3);
step (a2): first, converting starting material formic acid into formyl coenzyme A by catalysis with an enzyme having the function of catalyzing conversion of formic acid into formyl coenzyme A (the reaction is denoted by reaction 4), and then converting formyl coenzyme A into formaldehyde by catalysis with an enzyme having the function of catalyzing conversion of formyl coenzyme A into formaldehyde (the reaction is denoted by reaction 7); or
step (a3): first, converting starting material formic acid into formyl phosphate by catalysis with an enzyme having the function of catalyzing conversion of formic acid into formyl phosphate (the reaction is denoted by reaction 5), then converting formyl phosphate into formyl coenzyme A by catalysis with an enzyme having the function of catalyzing conversion of formyl phosphate into formyl coenzyme A (the reaction is denoted by reaction 6), and then converting formyl coenzyme A into formaldehyde by catalysis with an enzyme having the function of catalyzing conversion of formyl coenzyme A into formaldehyde (the reaction is denoted by reaction 7).

The present disclosure also provides a method (II) for the synthesis of starch, which comprises the following steps:
step 1): converting starting material methanol into amylose by catalysis with multiple enzymes; and
optional step 2): converting the amylose obtained in step 1) into amylopectin by catalysis with one or more enzymes.

According to an embodiment of the method (II) of the present disclosure, the enzyme used in step 1) is a combination of enzymes which catalyzes the synthesis of starch from methanol by multi-step reaction. For example, the enzyme may be the following enzyme combinations:
an enzyme combination (II-1-a): a combination of an enzyme having the function of catalyzing conversion of methanol into formaldehyde, an enzyme having the function of catalyzing conversion of formaldehyde into dihydroxyacetone, an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate, an enzyme having the function of catalyzing conversion of α-D-glucose-1-phosphate into adenosine diphosphate-α-D-glucose and
an enzyme having the function of catalyzing conversion of adenosine diphosphate-α-D-glucose into amylose;
an enzyme combination (II-1-b): a combination of an enzyme having the function of catalyzing conversion of methanol into formaldehyde, an enzyme having the function of catalyzing conversion of formaldehyde into dihydroxyacetone, an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate, an enzyme having the function of catalyzing conversion of α-D-glucose-1-phosphate into adenosine diphosphate-α-D-glucose and an enzyme having the function of catalyzing conversion of adenosine diphosphate-α-D-glucose into amylose;
an enzyme combination (II-1-c): a combination of an enzyme having the function of catalyzing conversion of methanol into formaldehyde, an enzyme having the function of catalyzing conversion of formaldehyde into dihydroxyacetone, an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate and an enzyme having the function of catalyzing conversion of α-D-glucose-1-phosphate into amylose; or
an enzyme combination (II-1-d): a combination of an enzyme having the function of catalyzing conversion of methanol into formaldehyde, an enzyme having the function of catalyzing conversion of formaldehyde into dihydroxyacetone, an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate and an enzyme having the function of catalyzing conversion of α-D-glucose-1-phosphate into amylose.

Specifically, step 1) may be performed according to the combination of reactions 2, 8, 9, 10, 13, 15, 16, 17 and 18, the combination of reactions 2, 8, 9, 10, 14, 15, 16, 17 and 18, the combination of reactions 2, 8, 9, 10, 11, 12, 15, 16, 17 and 18, the combination of reactions 2, 8, 9, 10, 13, 15, 16 and 19, the combination of reactions 2, 8, 9, 10, 14, 15, 16 and 19, or the combination of reactions 2, 8, 9, 10, 11, 12, 15, 16 and 19 described above.

According to an embodiment of the method (II) of the present disclosure, the enzyme used in step 2) is an enzyme having the function of catalyzing conversion of amylose into amylopectin. Specifically, step 2) may be performed according to reaction 20 described above.

The present disclosure also provides a method (III) for the synthesis of starch, which comprises the following steps:
step 1): converting starting material methanol into a compound D, namely dihydroxyacetone, by catalysis with one or more enzymes;
step 2): converting the dihydroxyacetone obtained in step 1) into amylose by catalysis with one or more enzymes; and
optional step 3): converting the amylose obtained in step 2) into amylopectin by catalysis with one or more enzymes.

According to an embodiment of the method (III) of the present disclosure, the enzyme used in step 1) is an enzyme combination (III-1): a combination of an enzyme having the function of catalyzing conversion of methanol into formaldehyde and an enzyme having the function of catalyzing conversion of formaldehyde into dihydroxyacetone.

Specifically, step 1) may be performed according to the combination of reactions 1 and 8 or the combination of reactions 2 and 8 described above.

According to an embodiment of the method (III) of the present disclosure, the enzyme used in step 2) is the following enzyme combinations:
an enzyme combination (III-2-a): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate, an enzyme having the function of catalyzing conversion of α-D-glucose-1-phosphate into adenosine diphosphate-α-D-glucose and
an enzyme having the function of catalyzing conversion of adenosine diphosphate-α-D-glucose into amylose;
an enzyme combination (III-2-b): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate, an enzyme having the function of catalyzing conversion of α-D-glucose-1-phosphate into adenosine diphosphate-α-D-glucose and an enzyme having the function of catalyzing conversion of adenosine diphosphate-α-D-glucose into amylose;
an enzyme combination (III-2-c): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate and an enzyme having the function of catalyzing conversion of α-D-glucose-1-phosphate into amylose; or
an enzyme combination (III-2-d): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate and an enzyme having the function of catalyzing conversion of α-D-glucose-1-phosphate into amylose.

Specifically, step 2) may be performed according to the combination of reactions 9, 10, 13, 15, 16, 17 and 18, the combination of reactions 9, 10, 14, 15, 16, 17 and 18, or the combination of reactions 9, 10, 11, 12, 15, 16, 17 and 18, the combination of reactions 9, 10, 13, 15, 16 and 19, the combination of reactions 9, 10, 14, 15, 16 and 19, or the combination of reactions 9, 10, 11, 12, 15, 16 and 19 described above.

According to an embodiment of the method (III) of the present disclosure, the enzyme used in step 3) is an enzyme having the function of catalyzing conversion of amylose into amylopectin. Specifically, step 3) may be performed according to reaction 20 described above.

The present disclosure also provides a method (IV) for the synthesis of starch, which comprises the following steps:
step 1): converting starting material formic acid into dihydroxyacetone by catalysis with one or more enzymes;
step 2): converting the dihydroxyacetone obtained in step 1) into amylose by catalysis with one or more enzymes; and
optional step 3): converting the amylose obtained in step 2) into amylopectin by catalysis with one or more enzymes.

According to an embodiment of the method (IV) of the present disclosure, the enzyme used in step 1) is an enzyme or a combination of enzymes which catalyzes conversion of formic acid into dihydroxyacetone by one-step or multi-step reaction. For example, the enzyme may be the following enzyme combinations:
an enzyme combination (IV-1-a): a combination of an enzyme having the function of catalyzing conversion of formic acid into formaldehyde and an enzyme having the function of catalyzing conversion of formaldehyde into dihydroxyacetone;
an enzyme combination (IV-1-b): a combination of an enzyme having the function of catalyzing conversion of formic acid into formyl coenzyme A, an enzyme having the function of catalyzing conversion of formyl coenzyme A into formaldehyde and an enzyme having the function of catalyzing conversion of formaldehyde into dihydroxyacetone; or
an enzyme combination (IV-1-c): a combination of an enzyme having the function of catalyzing conversion of formaldehyde into dihydroxyacetone, an enzyme having the function of catalyzing conversion of dihydroxyacetone into dihydroxyacetone phosphate and an enzyme having the function of catalyzing conversion of dihydroxyacetone phosphate into D-glyceraldehyde 3-phosphate.

Specifically, step 1) may be performed according to the combination of reactions 3 and 8, the combination of reactions 4, 7 and 8 or the combination of reactions 5, 6, 7 and 8 described above.

According to an embodiment of the method (IV) of the present disclosure, the enzyme used in step 2) is an enzyme or a combination of enzymes which catalyzes conversion of dihydroxyacetone into amylose by one-step or multi-step reaction. For example, the enzyme may be the following enzyme combinations:
an enzyme combination (IV-2-a): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate and an enzyme having the function of catalyzing conversion of α-D-glucose-1-phosphate into amylose;
an enzyme combination (IV-2-b): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate, an enzyme having the function of catalyzing conversion of α-D-glucose-1-phosphate into adenosine diphosphate-α-D-glucose and an enzyme having the function of catalyzing conversion of adenosine diphosphate-α-D-glucose into amylose;
an enzyme combination (IV-2-c): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate and an enzyme having the function of catalyzing conversion of α-D-glucose-1-phosphate into amylose; or
an enzyme combination (IV-2-d): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate, an enzyme having the function of catalyzing conversion of α-D-glucose-1-phosphate into adenosine diphosphate-α-D-glucose and an enzyme having the function of catalyzing conversion of adenosine diphosphate-α-D-glucose into amylose.

Specifically, step 2) may be performed according to the combination of reactions 9, 10, 13, 15, 16 and 19, the combination of reactions 9, 10, 14, 15, 16 and 19, the combination of reactions 9, 10, 13, 15, 16, 17 and 18, the combination of reactions 9, 10, 14, 15, 16, 17 and 18, the combination of reactions 9, 10, 11, 12, 15, 16 and 19, or the combination of reactions 9, 10, 11, 12, 15, 16, 17 and 18 described above.

According to an embodiment of the method (IV) of the present disclosure, the enzyme used in step 3) is an enzyme having the function of catalyzing conversion of amylose into amylopectin. Specifically, step 3) may be performed according to reaction 20 described above.

The present disclosure also provides a method (V) for the synthesis of starch, which comprises the following steps:
step 1): converting starting material formic acid into formaldehyde by catalysis with one or more enzymes;
step 2): converting the formaldehyde obtained in step 1) into dihydroxyacetone by catalysis with one or more enzymes;
step 3): converting the dihydroxyacetone obtained in step 2) into amylose by catalysis with one or more enzymes; and
optional step 4): converting the amylose obtained in step 3) into amylopectin by catalysis with one or more enzymes.

According to an embodiment of the method (V) of the present disclosure, the enzyme used in step 1) is a single enzyme having the function of catalyzing conversion of formic acid into formaldehyde, or the following enzyme combinations: an enzyme combination (V-1-a): an enzyme having the function of catalyzing conversion of formic acid into formyl coenzyme A and an enzyme having the function of catalyzing conversion of formyl coenzyme A into formaldehyde, or an enzyme combination (V-1-b): a combination of an enzyme having the function of catalyzing conversion of formic acid into formyl phosphate, an enzyme having the function of catalyzing conversion of formyl phosphate into formyl coenzyme A and an enzyme having the function of catalyzing conversion of formyl coenzyme A into formaldehyde.

Specifically, step 1) may be performed according to reaction 3, the combination of reactions 4 and 7 or the combination of reactions 5, 6 and 7.

According to an embodiment of the method (V) of the present disclosure, the enzyme used in step 2) is an enzyme having the function of catalyzing conversion of formaldehyde into dihydroxyacetone.

Specifically, step 2) may be performed according to reaction 8 described above.

According to an embodiment of the method (V) of the present disclosure, the enzyme used in step 3) is the following enzyme combinations:
an enzyme combination (V-3-a): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate and an enzyme having the function of catalyzing conversion of α-D-glucose-1-phosphate into amylose;
an enzyme combination (V-3-b): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate, an enzyme having the function of catalyzing conversion of α-D-glucose-1-phosphate into adenosine diphosphate-α-D-glucose and an enzyme having the function of catalyzing conversion of adenosine diphosphate-α-D-glucose into amylose;
an enzyme combination (V-3-c): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate and an enzyme having the function of catalyzing conversion of α-D-glucose-1-phosphate into amylose; or
an enzyme combination (V-3-d): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate, an enzyme having the function of catalyzing conversion of α-D-glucose-1-phosphate into adenosine diphosphate-α-D-glucose and an enzyme having the function of catalyzing conversion of adenosine diphosphate-α-D-glucose into amylose.

Specifically, step 3) may be performed according to the combination of reactions 9, 10, 13, 15, 16 and 19, the combination of reactions 9, 10, 14, 15, 16 and 19, the combination of reactions 9, 10, 13, 15, 16, 17 and 18, the combination of reactions 9, 10, 14, 15, 16, 17 and 18, the combination of reactions 9, 10, 11, 12, 15, 16 and 19, or the combination of reactions 9, 10, 11, 12, 15, 16, 17 and 18 described above.

According to an embodiment of the method (V) of the present disclosure, the enzyme used in step 4) is an enzyme having the function of catalyzing conversion of amylose into amylopectin. Specifically, step 4) may be performed according to reaction 20 described above.

The present disclosure also provides a method (VI) for the synthesis of starch, which comprises the following steps:
step 1): converting starting material formic acid into formaldehyde by catalysis with one or more enzymes;
step 2): converting the formaldehyde obtained in step 1) into a compound F, namely D-glyceraldehyde 3-phosphate, by catalysis with one or more enzymes;
step 3): converting the D-glyceraldehyde 3-phosphate obtained in step 2) into D-glucose-6-phosphate by catalysis with one or more enzymes;
step 4): converting the D-glucose-6-phosphate obtained in step 3) into amylose by catalysis with one or more enzymes; and
optional step 5): converting the amylose obtained in step 4) into amylopectin by catalysis with one or more enzymes.

According to an embodiment of the method (VI) of the present disclosure, the enzyme used in step 1) is an enzyme combination (VI-1): a combination of an enzyme having the function of catalyzing conversion of formic acid into formyl phosphate, an enzyme having the function of catalyzing conversion of formyl phosphate into formyl coenzyme A and an enzyme having the function of catalyzing conversion of formyl coenzyme A into formaldehyde.

Specifically, step 1) may be performed according to reaction 3, the combination of reactions 4 and 7 or the combination of reactions 5, 6 and 7.

According to an embodiment of the method (VI) of the present disclosure, the enzyme used in step 2) is an enzyme combination (VI-2): a combination of an enzyme having the function of catalyzing conversion of formaldehyde into dihydroxyacetone, an enzyme having the function of catalyzing conversion of dihydroxyacetone into dihydroxyacetone phosphate and an enzyme having the function of catalyzing conversion of dihydroxyacetone phosphate into D-glyceraldehyde 3-phosphate.

Specifically, step 2) may be performed according to the combination of reactions 8, 9 and 10 described above.

According to an embodiment of the method (VI) of the present disclosure, the enzyme used in step 3) is the following enzyme combinations:
an enzyme combination (VI-3-a): a combination of an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate and an enzyme having the function of catalyzing conversion of D-fructose-6-phosphate into D-glucose-6-phosphate; or
an enzyme combination (VI-3-b): a combination of an enzyme having the function of catalyzing conversion of D-glyceraldehyde 3-phosphate into D-fructose-6-phosphate and an enzyme having the function of catalyzing conversion of D-fructose-6-phosphate into D-glucose-6-phosphate.

Specifically, step 3) may be performed according to the combination of reactions 11, 12 and 15 described above, or step 3) may also be performed according to the combination of reactions 13 and 15 or the combination of reactions 14 and 15 described above.

According to an embodiment of the method (VI) of the present disclosure, the enzyme used in step 4) is the following enzyme combinations:
an enzyme combination (VI-4-a): a combination of an enzyme having the function of catalyzing conversion of D-glucose-6-phosphate into α-D-glucose-1-phosphate, and enzyme having the function of catalyzing conversion of α-D-glucose-1-phosphate into adenosine diphosphate-α-D-glucose and an enzyme having the function of catalyzing conversion of adenosine diphosphate-α-D-glucose into amylose; or
an enzyme combination (VI-4-b): a combination of an enzyme having the function of catalyzing conversion of D-glucose-6-phosphate into α-D-glucose-1-phosphate and an enzyme having the function of catalyzing conversion of α-D-glucose-1-phosphate into amylose.

Specifically, step 4) may be performed according to the combination of reactions 16, 17 and 18 described above, or step 4) may also be performed according to the combination of reactions 16 and 19 described above.

According to an embodiment of the method (VI) of the present disclosure, the enzyme used in step 5) is an enzyme having the function of catalyzing conversion of amylose into amylopectin. Specifically, step 5) may be performed according to reaction 20 described above.

The present disclosure also provides a method for the synthesis of dihydroxyacetone, which comprises steps of converting starting material methanol into dihydroxyacetone by catalysis with one or more enzymes.

According to an embodiment of the present disclosure, the method for the synthesis of dihydroxyacetone comprises the following steps:
step (1): converting starting material methanol into formaldehyde by catalysis with an enzyme having the function of catalyzing conversion of methanol into formaldehyde; and
step (2): converting the formaldehyde obtained in step (1) into dihydroxyacetone by catalysis with an enzyme having the function of catalyzing conversion of formaldehyde into dihydroxyacetone.

According to an embodiment of the present disclosure, the enzyme having the function of catalyzing conversion of methanol into formaldehyde in step (1) includes, but is not limited to, alcohol oxidase (AOX) or mutants thereof, cholesterol oxidase or mutants thereof, alcohol dehydrogenase (ADH) or mutants thereof, methanol dehydrogenase or mutants thereof, L-threonine-3-dehydrogenase or mutants thereof, cyclohexanol dehydrogenase or mutants thereof, or *n*-butanol dehydrogenase or mutants thereof.

The enzyme having the function of catalyzing conversion of formaldehyde into dihydroxyacetone in step (2) includes, but is not limited to, formolase (FLS) or mutants thereof (FLS-M), or glycolaldehyde synthase (GALS) or mutants thereof.

According to an embodiment of the present disclosure, steps (1) and (2) may be performed simultaneously or step by step.

According to an embodiment of the present disclosure, steps (1) and (2) are performed simultaneously; for example, the reaction system comprises substrate methanol, the enzyme having the function of catalyzing conversion of methanol into formaldehyde and the enzyme having the function of catalyzing conversion of formaldehyde into dihydroxyacetone. For example, the reaction system comprises substrate methanol, alcohol oxidase or alcohol dehydrogenase, and formolase. In addition, the reaction system may also optionally comprise an auxiliary enzyme such as catalase.

According to an embodiment of the present disclosure, the reaction system further comprises NaCl, Mg²⁺ and Zn²⁺ and the like.

According to an embodiment of the present disclosure, the reaction system has a pH of 6.5-8.5, such as 7-8; for example, the pH environment is provided by Hepes buffer.

According to the present disclosure, the steps, sub-steps or specific reactions (such as reaction 1 and reaction 2 and the like; the reactions are numbered as shown in the above Summary section or FIG. 1) of the method of the present disclosure can be performed step by step, or any adjacent two, three, four, five, six, seven or more steps, sub-steps or specific reactions can also be performed simultaneously, or all the steps or specific reactions can be performed simultaneously. The "adjacent" steps, sub-steps or particular reactions means that the product of the preceding step, sub-step or particular reaction can be used as the reactant of the subsequent step, sub-step or particular reaction, and then the two steps, sub-steps or particular reactions can be referred to as being "adjacent". The "performed step by step" means that after the preceding reaction is completed, the product is or is not purified, and then the enzyme or required component for the subsequent reaction is fed to carry out the subsequent reaction. The "performed simultaneously" means that the enzymes and substrates for the reactions involved in the catalysis are fed together into a reactor at the beginning of the reactions and are reacted. For example, the reactions below linked by "-" mean that they are performed simultaneously: reactions 2-8, reactions 4-7, reactions 5-6-7, reactions 5-6-7-8, reactions 8-9-10, reactions 11-12-15, reactions 13-15, reactions 14-15, reactions 16-17-18, reactions 16-19, reactions 9-10-11-12-15-16-17-18, reactions 9-10-13-15-16-17-18, reactions 9-10-14-15-16-17-18, reactions 9-10-13-15-16-19, reactions 9-10-11-12-15-16-17-18-20, and reactions 2-8-9-10-11-12-15-16-17-18. For example, "reactions 2-8" means that reaction 2 and reaction 8 are performed simultaneously, "reactions 4-7" means that reaction 4 and reaction 7 are performed simultaneously, and so on.

In the context of the present disclosure, "the combination of reactions A and B..." means that the conversion of reactants into products is achieved by carrying out the reactions in the combination, which can be performed step by step or simultaneously. For example, "the combination of reactions 4 and 7" means that the conversion of formic acid into formaldehyde is achieved by carrying out reaction 4 and reaction 7, wherein reaction 4 and reaction 7 can be performed step by step or simultaneously; "the combination of reactions 9, 10, 11, 12, 15, 16 and 19" means that the conversion of dihydroxyacetone into amylose is achieved by carrying out reactions 9, 10, 11, 12, 15, 16 and 19, wherein adjacent two, three, four, five, six or seven of reactions 9, 10, 11, 12, 15, 16 and 19 can be performed step by step or simultaneously; and so on.

According to the present disclosure, different reactions in which the starting materials and the products are identical in the same step or sub-step of each method are interchangeable.

According to the present disclosure, different reactions in different steps or sub-steps of each method can be combined at will, so long as the subsequent reaction takes the product of the preceding reaction as the starting material and the synthesis of the final product starch can be achieved by combining them.

For example, compound 1 (i.e., amylose) can be achieved by using dihydroxyacetone as a starting material and carrying out the following combinations of reactions (the reactions are numbered as shown in FIG. 1): the combination of reactions 9, 10, 11, 12, 15, 16, 17 and 18, the combinations of reactions 9, 10, 13, 15, 16, 17 and 18, the combination of reactions 9, 10, 14, 15, 16, 17 and 18, the combination of reactions 9, 10, 11, 12, 15, 16 and 19, the combination of reactions 9, 10, 13, 15, 16 and 19, and the combination of reactions 9, 10, 14, 15, 16 and 19.

Compound 2 (i.e., amylopectin) can be achieved by using dihydroxyacetone as a starting material and carrying out the following combinations of reactions (the reactions are numbered as shown in FIG. 1): the combination of reactions 9, 10, 11, 12, 15, 16, 17, 18 and 20, the combinations of reactions 9, 10, 13, 15, 16, 17, 18 and 20, the combination of reactions 9, 10, 14, 15, 16, 17, 18 and 20, the combination of reactions 9, 10, 11, 12, 15, 16, 19 and 20, the combination of reactions 9, 10, 13, 15, 16, 19 and 20, and the combination of reactions 9, 10, 14, 15, 16, 19 and 20.

Compound 1 (i.e., amylose) can be achieved by using formaldehyde as a starting material and carrying out the following combinations of reactions (the reactions are numbered as shown in FIG. 1): the combination of reactions 8, 9, 10, 11, 12, 15, 16, 17 and 18, the combinations of reactions 8, 9, 10, 13, 15, 16, 17 and 18, the combination of reactions 8, 9, 10, 14, 15, 16, 17 and 18, the combination of reactions 8, 9, 10, 11, 12, 15, 16 and 19, the combination of reactions 8, 9, 10, 13, 15, 16 and 19, and the combination of reactions 8, 9, 10, 14, 15, 16 and 19.

Compound 2 (i.e., amylopectin) can be achieved by using formaldehyde as a starting material and carrying out the following combinations of reactions (the reactions are numbered as shown in FIG. 1): the combination of reactions 8, 9, 10, 11, 12, 15, 16, 17, 18 and 20, the combinations of reactions 8, 9, 10, 13, 15, 16, 17, 18 and 20, the combination of reactions 8, 9, 10, 14, 15, 16, 17, 18 and 20, the combination of reactions 8, 9, 10, 11, 12, 15, 16, 19 and 20, the combination of reactions 8, 9, 10, 13, 15, 16, 19 and 20, and the combination of reactions 8, 9, 10, 14, 15, 16, 19 and 20.

Compound 1 (i.e. amylose) can be achieved by using methanol or formic acid as a starting material and carrying out the following combinations of reactions (the reactions are numbered as shown in FIG. 1): the combination of reactions 1, 8, 9, 10, 11, 12, 15, 16, 17 and 18, the combination of reactions 2, 8, 9, 10, 11, 12, 15, 16, 17 and 18, the combination of reactions 3, 8, 9, 10, 11, 12, 15, 16, 17 and 18, the combination of reactions 4, 7, 8, 9, 10, 11, 12, 15, 16, 17 and 18, the combination of reactions 5, 6, 7, 8, 9, 10, 11, 12, 15, 16, 17 and 18, the combination of reactions 1, 8, 9, 10, 13, 15, 16, 17 and 18, the combination of reactions 2, 8, 9, 10, 13, 15, 16, 17 and 18, the combination of reactions 3, 8, 9, 10, 13, 15, 16, 17 and 18, the combination of reactions 4, 7, 8, 9, 10, 13, 15, 16, 17 and 18, the combination of reactions 5, 6, 7, 8, 9, 10, 13, 15, 16, 17 and 18, the combination of reactions 1, 8, 9, 10, 14, 15, 16, 17 and 18, the combination of reactions 2, 8, 9, 10, 14, 15, 16, 17 and 18, the combination of reactions 3, 8, 9, 10, 14, 15, 16, 17 and 18, the combination of reactions 4, 7, 8, 9, 10, 14, 15, 16, 17 and 18, the combination of reactions 5, 6, 7, 8, 9, 10, 14, 15, 16, 17 and 18, the combination of reactions 1, 8, 9, 10, 11, 12, 15, 16 and 19, the combination of reactions 2, 8, 9, 10, 11, 12, 15, 16 and 19, the combination of reactions 3, 8, 9, 10, 11, 12, 15, 16 and 19, the combination of reactions 4, 7, 8, 9, 10, 11, 12, 15, 16 and 19, the combination of reactions 5, 6, 7, 8, 9, 10, 11, 12, 15, 16 and 19, the combination of reactions 1, 8, 9, 10, 13, 15, 16 and 19, the combination of reactions 2, 8, 9, 10, 13, 15, 16 and 19, the combination of reactions 3, 8, 9, 10, 13, 15, 16 and 19, the combination of reactions 4, 7, 8, 9, 10, 13, 15, 16 and 19, the combination of reactions 5, 6, 7, 8, 9, 10, 13, 15, 16 and 19, the combination of reactions 1, 8, 9, 10, 14, 15, 16 and 19, the combination of reactions 2, 8, 9, 10, 14, 15, 16 and 19, the combination of reactions 3, 8, 9, 10, 14, 15, 16 and 19, the combination of reactions 4, 7, 8, 9, 10, 14, 15, 16 and 19, and the combination of reactions 5, 6, 7, 8, 9, 10, 14, 15, 16 and 19.

Compound 2 (i.e. amylopectin) can be achieved by using methanol or formic acid as a starting material and carrying out the following combinations of reactions (the reactions are numbered as shown in FIG. 1): the combination of reactions 1, 8, 9, 10, 11, 12, 15, 16, 17, 18 and 20, the combination of reactions 2, 8, 9, 10, 11, 12, 15, 16, 17, 18 and 20, the combination of reactions 3, 8, 9, 10, 11, 12, 15, 16, 17, 18 and 20, the combination of reactions 4, 7, 8, 9, 10, 11, 12, 15, 16, 17, 18 and 20, the combination of reactions 5, 6, 7, 8, 9, 10, 11, 12, 15, 16, 17, 18 and 20, the combination of reactions 1, 8, 9, 10, 13, 15, 16, 17, 18 and 20, the combination of reactions 2, 8, 9, 10, 13, 15, 16, 17, 18 and 20, the combination of reactions 3, 8, 9, 10, 13, 15, 16, 17, 18 and 20, the combination of reactions 4, 7, 8, 9, 10, 13, 15, 16, 17, 18 and 20, the combination of reactions 5, 6, 7, 8, 9, 10, 13, 15, 16, 17, 18 and 20, the combination of reactions 1, 8, 9, 10, 14, 15, 16, 17, 18 and 20, the combination of reactions 2, 8, 9, 10, 14, 15, 16, 17, 18 and 20, the combination of reactions 3, 8, 9, 10, 14, 15, 16, 17, 18 and 20, the combination of reactions 4, 7, 8, 9, 10, 14, 15, 16, 17, 18 and 20, the combination of reactions 5, 6, 7, 8, 9, 10, 14, 15, 16, 17, 18 and 20, the combination of reactions 1, 8, 9, 10, 11, 12, 15, 16, 19 and 20, the combination of reactions 2, 8, 9, 10, 11, 12, 15, 16, 19 and 20, the combination of reactions 3, 8, 9, 10, 11, 12, 15, 16, 19 and 20, the combination of reactions 4, 7, 8, 9, 10, 11, 12, 15, 16, 19 and 20, the combination of reactions 5, 6, 7, 8, 9, 10, 11, 12, 15, 16, 19 and 20, the combination of reactions 1, 8, 9, 10, 13, 15, 16, 19 and 20, the combination of reactions 2, 8, 9, 10, 13, 15, 16, 19 and 20, the combination of reactions 3, 8, 9, 10, 13, 15, 16, 19 and 20, the combination of reactions 4, 7, 8, 9, 10, 13, 15, 16, 19 and 20, the combination of reactions 5, 6, 7, 8, 9, 10, 13, 15, 16, 19 and 20, the combination of reactions 1, 8, 9, 10, 14, 15, 16, 19 and 20, the combination of reactions 2, 8, 9, 10, 14, 15, 16, 19 and 20, the combination of reactions 3, 8, 9, 10, 14, 15, 16, 19 and 20, the combination of reactions 4, 7, 8, 9, 10, 14, 15, 16, 19 and 20, and the combination of reactions 5, 6, 7, 8, 9, 10, 14, 15, 16, 19 and 20.

When an enzyme used in the context of the present application is described with such an expression as "an enzyme having the function of catalyzing conversion of substance A into substance B", it is meant that the enzyme can catalyze the reaction of converting substance A into substance B. The reaction can be a one-step reaction or multi-step reaction, and the enzyme can be one that is required by any step of reaction in the production of substance B from substance A. Thus, the enzyme can be a single enzyme which catalyzes the one-step reaction or a combination of enzymes which catalyzes one or more steps of the multi-step reaction. The amino acid sequences and origins of the enzymes having the catalytic functions are not particularly limited so long as they can fulfill the catalytic functions. Specifically, "an enzyme having the function of catalyzing conversion of methanol into formaldehyde" refers to an enzyme capable of catalyzing the reaction of converting methanol into formaldehyde, including but not limited to alcohol oxidase (AOX, EC 1.1.3.13) or mutants thereof, cholesterol oxidase (EC 1.1.3.6) or mutants thereof, alcohol dehydrogenase (ADH, EC 1.1.1.1; EC 1.1.1.2; EC 1.1.1.71; EC 1.1.2.8) or mutants thereof, methanol dehydrogenase (EC 1.1.1.244; EC 1.1.2.7; EC 1.1.2.B2) or mutants thereof, L-threonine-3-dehydrogenase (EC 1.1.1.103) or mutants thereof, cyclohexanol dehydrogenase (EC 1.1.1.245) or mutants thereof, or n-butanol dehydrogenase (EC 1.1.2.9) or mutants thereof. These enzymes may be derived from, but not limited to, various species such as *Pichia pastoris, Candida, Streptomyces, Corynebacterium glutamicum, Escherichia coli, Rhodococcus* and *Thauera* and the like. "An enzyme having the function of catalyzing conversion of formic acid into formaldehyde" refers to an enzyme capable of catalyzing the reaction of converting formic acid into formaldehyde, including but not limited to aldehyde dehydrogenase (ADH, EC 1.2.1.3; EC 1.2.1.4; EC 1.2.1.5) or mutants thereof. These enzymes may be derived from, but not limited to, various species such as *Burkholderia cepacia, Pseudomonas adaceae, Acetobacter aceti* and *Saccharomyces* and the like. "An enzyme having the function of catalyzing conversion of formic acid into formyl coenzyme A" refers to an enzyme capable of catalyzing the reaction of converting formic acid into formyl coenzyme A, including but not limited to acetyl-coenzyme A synthetase (ACS, EC 6.2.1.1) or mutants thereof. These enzymes may be derived from, but not limited to, various species such as *Escherichia coli, Salmonella, Pseudomonas adaceae, Moorella* and *Pyrobaculum* and the like. "An enzyme having the function of catalyzing conversion of formyl coenzyme A into formaldehyde" refers to an enzyme capable of catalyzing the reaction of converting formyl coenzyme A into formaldehyde, including but not limited to acetaldehyde dehydrogenase (ACDH, EC 1.2.1.10) or mutants thereof. These enzymes may be derived from, but not limited to, various species such as *Listeria monocytogenes, Pseudomonas adaceae, Acinetobacter* and *Giardia duodenalis* and the like. "An enzyme having the function of catalyzing conversion of formic acid into formyl phosphate" refers to an enzyme capable of catalyzing the reaction of converting formic acid into formyl phosphate, including but not limited to acetate kinase (ACKA, EC 2.7.2.1) or mutants thereof. These enzymes may be derived from, but not limited to, various species such as *Escherichia coli, Salmonella, Clostridium* and *Methanosarcina* and the like. "An enzyme having the function of catalyzing conversion of formyl phosphate into formyl coenzyme A" refers to an enzyme capable of catalyzing the reaction of converting formyl phosphate into formyl coenzyme A, including but not limited to phosphate acetyltransferase (PTA, EC 2.3.1.8) or mutants thereof. These enzymes may be derived from, but not limited to, various species such as *Escherichia coli, Clostridium, Thermotoga* and *Methanosarcina* and the like. "An enzyme having the function of catalyzing conversion of formaldehyde into dihydroxyacetone" refers to an enzyme capable of catalyzing the reaction of converting formaldehyde into dihydroxyacetone, including but not limited to formolase (FLS) or mutants thereof (FLS-M), or glycolaldehyde synthase (GALS) or mutants thereof. "An enzyme having the function of catalyzing conversion of dihydroxyacetone into dihydroxyacetone phosphate" refers to an enzyme capable of catalyzing the reaction of converting dihydroxyacetone into dihydroxyacetone phosphate, including but not limited to triose kinase (EC 2.7.1.28) or mutants thereof, dihydroxyacetone kinase (DAK, EC 2.7.1.29) or mutants thereof, or glycerol kinase (EC 2.7.1.30) or mutants thereof. These enzymes may be derived from, but not limited to, various species such as *Escherichia coli, Clostridium, Saccharomyces cerevisiae, Pichia pastoris, Citrobacter, Bacillus thermophilus* and *Homo sapiens* and the like. "An enzyme having the function of catalyzing conversion of dihydroxyacetone phosphate into D-glyceraldehyde 3-phosphate" refers to an enzyme capable of catalyzing the reaction of converting dihydroxyacetone phosphate into D-glyceraldehyde 3-phosphate, including but not limited to triose phosphate isomerase (TPI, EC 5.3.1.1) or mutants thereof. These enzymes may be derived from, but not limited to, various species such as *Escherichia coli, Saccharomyces, Staphylococcus aureus, Tubercle bacillus, Arabidopsis thaliana* and *Homo sapiens* and the like.

"An enzyme having the function of catalyzing conversion of D-glyceraldehyde 3-phosphate into D-fructose-1,6-bisphosphate" refers to an enzyme capable of catalyzing the reaction of converting D-glyceraldehyde 3-phosphate and dihydroxyacetone phosphate into D-fructose-1,6-diphosphate, including but not limited to fructose-bisphosphate aldolase (FBA, EC 4.1.2.13) or mutants thereof. These enzymes may be derived from, but not limited to, various species such as *Escherichia coli, Saccharomyces, Bacillus,* algae, *Clostridium* and *Homo sapiens* and the like. "An enzyme having the function of catalyzing conversion of D-fructose-1,6-bisphosphate into D-fructose-6-phosphate" refers to an enzyme capable of catalyzing the reaction of converting D-fructose-1,6-bisphosphate into D-fructose-6-phosphate, including but not limited to fructose-bisphosphatase (FBP, EC 3.1.3.11) or mutants thereof (FBP-M), inositol phosphatase (EC 3.1.3.25) or mutants thereof, or N-acylneuraminate-9-phosphatase (EC 3.1.3.29) or mutants thereof. These enzymes may be derived from, but not limited to, various species such as *Escherichia coli, Corynebacterium glutamicum, Saccharomyces, Bacillus,* algae, *Clostridium* and *Homo sapiens* and the like. "An enzyme having the function of catalyzing conversion of D-glyceraldehyde 3-phosphate into D-fructose-6-phosphate" refers to an enzyme capable of catalyzing the reaction of D-glyceraldehyde 3-phosphate and dihydroxyacetone or dihydroxyacetone phosphate into D-fructose-6-phosphate, including but not limited to fructose 6-phosphate aldolase (FSA) or mutants thereof, transaldolase (EC 2.2.1.2) or mutants thereof, or fructose 6-phosphate aldolase phosphatase (FBAP, EC 4.1.2.13 & 3.1.3.11) or mutants thereof. These enzymes may be derived from, but not limited to, various species such as *Escherichia coli, Saccharomyces,* algae, archaea and *Homo sapiens* and the like. "An enzyme having the function of catalyzing conversion of D-fructose-6-phosphate into D-glucose-6-phosphate" refers to an enzyme capable of catalyzing the reaction of converting D-fructose-6-phosphate into D-glucose-6-phosphate, including but not limited to glucose phosphate isomerase (PGI, EC 5.3.1.9) or mutants thereof, or mannose phosphate isomerase (EC 5.3.1.8) or mutants thereof. These enzymes may be derived from, but not limited to, various species such as *Escherichia coli, Saccharomyces,* methanogens, *Tubercle bacillus, Salmonella* and *Rhizobium* and the like. "An enzyme having the function of catalyzing conversion of D-glucose-6-phosphate into α-D-glucose-1-phosphate" refers to an enzyme capable of catalyzing the reaction of converting D-glucose-6-phosphate into α-D-glucose-1-phosphate, including but not limited to phosphohexose phosphate mutase (PGM, EC 5.4.2.2) or mutants thereof, or phosphoacetylglucosamine mutase (EC 5.4.2.3) or mutants thereof. These enzymes may be derived from, but not limited to, various species such as *Escherichia coli, Lactococcus lactis, Saccharomyces, Bacillus, Pseudomonas, Salmonella,* corn and *Homo sapiens* and the like. "An enzyme having the function of catalyzing conversion of α-D-glucose-1-phosphate into adenosine diphosphate-α-D-glucose" refers to an enzyme capable of catalyzing the reaction of converting α-D-glucose-1-phosphate into adenosine diphosphate-α-D-glucose, including but not limited to glucose-1-phosphate adenylyltransferase (GlgC, EC 2.7.7.27) or mutants thereof. These enzymes may be derived from, but not limited to, various species such as *Escherichia coli, Lactococcus lactis, Saccharomyces, Bacillus, Pseudomonas, Agrobacterium tumefaciens,* corn and potato and the like. "An enzyme having the function of catalyzing conversion of adenosine diphosphate-α-D-glucose into amylose" refers to an enzyme capable of catalyzing the reaction of converting adenosine diphosphate-α-D-glucose into amylose, including but not limited to starch synthase (GlgA, EC 2.4.1.21) or mutants thereof. These enzymes may be derived from, but not limited to, various species such as *Escherichia coli, Saccharomyces, Bacillus,* algae, corn and potato and the like. "An enzyme having the function of catalyzing conversion of α-D-glucose-1-phosphate into amylose" refers to an enzyme capable of catalyzing the reaction of converting α-D-glucose-1-phosphate into amylose, including but not limited to starch phosphorylase (αGP, EC 2.4.1.1) alone or mutants thereof, or a combination of glucose-1-phosphate adenylyltransferase (GlgC, EC 2.7.7.27) or mutants thereof and starch synthase (GlgA, EC 2.4.1.21) or mutants thereof. These enzymes may be derived from, but not limited to, various species such as *Escherichia coli, Saccharomyces, Bacillus,* algae, corn and potato and the like. "An enzyme having the function of catalyzing conversion of amylose into amylopectin" refers to an enzyme capable of catalyzing the reaction of converting amylose into amylopectin, including but not limited to 1,4-α-D-glucan branching enzyme (GlgB, EC 2.4.1.18) or mutants thereof. These enzymes may be derived from, but not limited to, various species such as *Escherichia coli, Bacillus, Vibrio, Saccharomyces,* corn and potato and the like.

The enzymes or mutants thereof used in the context of the present disclosure may be in the form of crude enzyme liquids, lyophilized powders of crude enzyme liquids, pure enzymes or whole cells. The crude enzyme liquids, the lyophilized powders of crude enzyme liquids, the pure enzymes or the whole cells are commercially available or can be prepared according to known methods in the literature or conventional methods in the art. For example, the crude enzyme liquids, the lyophilized powders of crude enzyme liquids and the pure enzymes are all prepared according to a method comprising the following steps: expressing the enzymes or mutants thereof in host cells to obtain recombinant cells, and lysing the recombinant cells to obtain the crude enzyme liquids, the lyophilized powders of crude enzyme liquids and the pure enzymes; the whole cells were all prepared according to a method comprising the following step: expressing the enzymes or mutants thereof in host cells to obtain recombinant cells, namely the whole cells.

### Beneficial Effects

With the methods of the present disclosure, the artificial biosynthesis of starch from simple compounds such as dihydroxyacetone, formaldehyde, formic acid and methanol and the like can be achieved. The total artificial biosynthesis of starch from starting material carbon dioxide can even be achieved by coupling the methods with relatively mature methods at present such as chemical reduction of carbon dioxide. The synthesis of natural starch needs to go through the Calvin cycle and involves a total of 21-22 reactions, whereas the methods of the present disclosure involve only 9-12 reactions-nearly half of the reactions are reduced. Moreover, the methods of the present disclosure do not involve the use of the well-known rate-limiting enzyme Rubisco and thus have more advantages with respect to synthesis rate, so the synthesis period can be greatly shortened. In addition, compared to agricultural planting which can utilize only low concentrations of carbon dioxide in the air and low-energy-density solar energy, the methods of the present disclosure can utilize high concentrations of carbon dioxide and high-energy-density electric energy and hydrogen energy and are more suitable for industrial production modes. With these methods, the production period can be reduced from several months, for agricultural planting, to several days, and the agricultural consumption of land and water is expected to be greatly reduced, which is of great significance for solving the problem that China has a large population and has limited arable land and fresh water resources. In addition, these features also make the methods of the present disclosure to be suitable for realizing circular supply of starch in closed spaces such as spacecraft and space stations and the like, which is of great significance for the space exploration policies in China.

### BRIEF DESCRIPTION OF THE DRAWINGS

FIG. 1 shows a synthesis pathway for the synthesis of compound 1, namely amylose, and compound 2, namely amylopectin, from starting material methanol or formic acid.
FIG. 2 shows a schematic of the structure of compound 1.
FIG. 3 shows a schematic of the structure of compound 2.
FIG. 4 shows the yield of the synthesis pathway from methanol to compound D (i.e. dihydroxyacetone).
FIG. 5 shows full-wavelength scanning spectra before and after the reaction of the pathway from compound 1 (i.e. amylose) to compound 2 (i.e. amylopectin).
FIG. 6 shows the yields of different pathways from compound D to compound 1.
FIG. 7 shows the yield of a different pathway from compound D to compound 2.
FIG. 8 shows the yield of the synthesis pathway from methanol to compound 1.

### DETAILED DESCRIPTION

The synthesis method disclosed herein will be further illustrated in detail with reference to the following specific examples. It should be understood that the following embodiments are merely exemplary illustration and explanation of the present disclosure, and should not be construed as limiting the protection scope of the present disclosure. All techniques implemented based on the content of the present disclosure described above are encompassed within the protection scope of the present disclosure.

Unless otherwise stated, the starting materials and reagents used in the following examples are all commercially available products or can be prepared using known methods.

The catalysts (enzymes) used for the reactions involved in the examples are shown in Table 1 below unless otherwise stated:

**Table 1. The catalysts used for the reactions involved in the examples**

| **Reaction** | **Full name of catalyst** | **Abbreviation** | **Host** | **Origin/Gene information** |
|---|---|---|---|---|
| 1 | Alcohol dehydrogenase | ADH | *Bacillus methanolicus* | Sigma (A7011) |
| 2 | Alcohol oxidase | AOX | *Pichia pastoris* | Sigma (A2404) |
| 3 | Aldehyde dehydrogenase | FADH | *Burkholderia multivorans* | BAG46824.1 |
| 4 | Acetyl-coenzyme A synthetase | ACS | *Escherichia coli* | AAC77039.1 |
| 5 | Acetate kinase | ACKA | *Escherichia coli* | AAC75356.1 |
| 6 | Phosphate acetyltransferase | PTA | *Escherichia coli* | AAC75511.1 |
| 7 | Acetaldehyde dehydrogenase | ACDH | *Listeria monocytogenes* | CAC99712.1 |
| 8 | Formolase | FLS | *Pseudomonas putida* | # |
| 9 | Dihydroxyacetone kinase | DAK | *Pichia pastoris* | AAC39490.1 |
| 10 | Triose phosphate isomerase | TPI | *Escherichia coli* | AAC76901.1 |
| 11 | Fructose-bisphosphate aldolase | FBA | *Escherichia coli* | AAC75158.2 |
| 12 | Fructose-bisphosphatas e | FBP | *Escherichia coli* | AAC77189.1 |
| 13 | Fructose 6-phosphate aldolase | FSA | *Escherichia coli* | AAC73912.2 |
| 14 | Fructose 6-phosphate aldolase phosphatase | FBAP | *Cenarchaeum symbiosum* | ABK77197.1 |
| 15 | Glucose phosphate isomerase | PGI | *Escherichia coli* | AAC76995.1 |
| 16 | Phosphohexose phosphate mutase | PGM | *Lactococcus Lactis* | CAL97144.1 |
| 17 | Glucose-1 -phosphate adenylyltransferase | GlgC | *Escherichia coli* | AAC76455.1 |
| 18 | Starch synthase | GlgA | *Escherichia coli* | AAC76454.1 |
| 19 | Starch phosphorylase | αGP | *Hordeum vulgare* | BAK00834.1 |
| | 1,4-α-D-Glucan 20 branching enzyme | GlgB | *Vibrio vulnificus* | ADV88080.1 |
| Auxiliary enzyme | Catalase | CAT | *Bacillus subtilis* 168 | CAB 12710.2 |
| | Formate dehydrogenase | FDH | *Thiobacillus* sp. KNK65MA | BAC92737.1 |
| | Inorganic pyrophosphatase | PPase | *Escherichia coli* | AAC77183.1 |

| | | | | |
|---|---|---|---|---|
| #: FLS gene information is available in the document (Siegel, J.B., et al., Computational protein design enables a novel one-carbon assimilation pathway. Proc Natl Acad Sci USA, 2015. 112(12): p. 3704-9.). | | | | |

Alcohol dehydrogenase and alcohol oxidase were purchased from Sigma (https://www.sigmaaldrich.com/china-mainland.html). Aldehyde dehydrogenase, acetyl-formyl coenzyme A synthetase, acetate kinase, phosphate acetyltransferase, acetaldehyde dehydrogenase, catalase, formate dehydrogenase and inorganic pyrophosphatase were obtained by PCR or gene synthesis and were cloned into vectors pET20b, pET21b, pET26b and pET28a (Novagen, Madison, WI) by simple cloning (You, C., et al. (2012). "Simple Cloning via Direct Transformation of PCR Product (DNA Multimer) to Escherichia coli and Bacillus subtilis." Appl. Environ. Microbiol. 78(5): 1593-1595.) to obtain corresponding expression vectors pET28a-FADH, pET21b-ACS, pET28a-ACKA, pET28a-PTA, pET21b-ACDH, pET26b-CAT, pET20b-FDH and pET21b-PPase. These eight plasmids were all transformed into *Escherichia coli* expression system BL21(DE3) (Invitrogen, Carlsbad, CA), and the proteins were expressed and purified.

Formolase, dihydroxyacetone kinase and triose phosphate isomerase were obtained by PCR or gene synthesis and were cloned into pET21a, pET21b and pET28a vectors (Novagen, Madison, WI) respectively by simple cloning (You, C., et al. (2012). Appl. Environ. Microbiol. 78(5): 1593-1595.) to obtain corresponding expression vectors pET21a-FLS, pET21b-TPI and pET28a-DAK. These three plasmids were all transformed into *Escherichia coli* expression system BL21(DE3) (Invitrogen, Carlsbad, CA), and the proteins were expressed and purified.

Fructose-bisphosphate aldolase, fructose-bisphosphatase, fructose 6-phosphate aldolase, fructose 6-phosphate aldolase phosphatase and glucose phosphate isomerase were obtained by PCR or gene synthesis and were cloned into pET21b vector (Novagen, Madison, WI) by simple cloning (You, C., et al. (2012). Appl. Environ. Microbiol. 78(5): 1593-1595.) to obtain corresponding expression vectors pET21b-FBA, pET21b-FBP, pET21b-FSA, pET21b-FBAP and pET21b-PGI. These five plasmids were all transformed into *Escherichia coli* expression system BL21(DE3) (Invitrogen, Carlsbad, CA), and the proteins were expressed and purified.

Phosphohexose phosphate mutase, glucose-1-phosphate adenylyltransferase, starch synthase and starch phosphorylase were obtained by PCR or gene synthesis and were cloned into pET20b and pET21b vectors (Novagen, Madison, WI) by simple cloning (You, C., et al. (2012). Appl. Environ. Microbiol. 78(5): 1593-1595.) to obtain corresponding expression vectors pET21b-PGM, pET21b-GlgC, pET21b-GlgA and pET20b-αGP. These four plasmids were all transformed into *Escherichia coli* expression system BL21(DE3) (Invitrogen, Carlsbad, CA), and the proteins were expressed and purified. 1,4-α-D-Glucan branching enzyme was obtained by PCR or gene synthesis and was cloned into pET28a vectors (Novagen, Madison, WI) by simple cloning (You, C., et al. (2012). Appl. Environ. Microbiol. 78(5): 1593-1595.) to obtain corresponding expression vector pET28a-GlgB. This plasmid was transformed into *Escherichia coli* expression system BL21(DE3) (Invitrogen, Carlsbad, CA), and the protein was expressed and purified.

### Example 1. Synthesis of Compound C, Namely Formaldehyde, from Formic Acid or Methanol

There were five pathways to achieve the conversion of formic acid or methanol into compound C: Pathway 1 (from methanol to formaldehyde), Pathway 2 (from methanol to formaldehyde), Pathway 3 (from formic acid to formaldehyde), Pathway 4-7 (from formic acid to formyl coenzyme A to formaldehyde) and Pathway 5-6-7 (from formic acid to formyl phosphate, then to formyl coenzyme A, and then to formaldehyde) (the reactions were numbered as shown in FIG. 1). First, the catalysts (i.e., enzymes) capable of catalyzing the chemical reactions of the pathways were selected (see Table 1); however, enzymes having the corresponding catalytic functions are not limited to those listed in Table 1. Then different catalysts were combined according to the pathways, and corresponding reaction systems were established. After the reactions were performed for a period of time, the yield of formaldehyde was determined.

The yield of compound C was determined as follows: to 200 µL of water was added 50 µL of test solution (was properly diluted)and then 25 µL of acetylacetone solution (100 mL of the acetylacetone solution comprised 0.5 mL of acetyl acetone, 50 g of ammonium acetate and 6 mL of glacial acetic acid); the mixture was reacted at 60 °C for 15 min and then centrifuged; 200 µL of the supernatant was collected and assayed for the OD414 value, and the compound C content was calculated from the formaldehyde standard curve.
**Pathway 1:** The reaction system comprised 100 mM Tris buffer with pH of 8.5, 100 mM NaCl, 5 mM Mg²⁺, 10 µM Zn²⁺, 3.5 mg/mL ADH, 100 mM NAD⁺ and 1 M methanol. The reaction was performed for 3 h, and the yield of formaldehyde was 0.27 mM.
**Pathway 2:** The reaction system comprised 100 mM Hepes buffer with pH of 7.5, 100 mM NaCl, 5 mM Mg²⁺, 10 µM Zn²⁺, 1 U/mL AOX, 300 U/mL CAT (auxiliary enzyme for eliminating hydrogen peroxide generated by AOX) and 20 mM methanol. The reaction was performed for 0.5 h, and the yield of formaldehyde was 12 mM.
**Pathway 3:** The reaction system comprised 100 mM Hepes buffer with pH of 7.5, 100 mM NaCl, 5 mM Mg²⁺, 10 µM Zn²⁺, 4 mg/mL FADH, 100 mM NADH and 250 mM sodium formate. The reaction was performed for 3 h, and the yield of formaldehyde was 0.1 mM.
**Pathway 4-7:** The reaction system comprised 100 mM Hepes buffer with pH of 7.5, 100 mM NaCl, 5 mM Mg²⁺, 10 µM Zn²⁺, 2 mM ATP, 1.5 mM NADH, 0.1 mM CoA, 3.7 mg/mL ACS, 0.2 mg/mL ACDH, 0.024 mg/mL FDH (auxiliary enzyme for regenerating NADH), 0.1 mg/mL PPase (auxiliary enzyme for hydrolyzing pyrophosphoric acid) and 50 mM sodium formate. The reaction was performed for 1 h, and the yield of formaldehyde was 0.6 mM.
**Pathway 5-6-7:** The reaction system comprised 100 mM Hepes buffer with pH of 7.5, 100 mM NaCl, 5 mM Mg²⁺, 10 µM Zn²⁺, 0.5 mM NADH, 10 mM ATP, 0.1 mM CoA, 2 mM β-mercaptoethanol, 0.24 mg/mL ACKA, 1.2 mg/mL PTA, 0.2 mg/mL ACDH, 0.024 mg/mL FDH (auxiliary enzyme for regenerating NADH) and 50 mM sodium formate. The reaction was performed for 1 h, and the yield of formaldehyde was 2 mM.

### Example 2. Synthesis of Compound D, Namely Dihydroxyacetone, from Methanol

The synthesis of compound D from methanol can be achieved via the pathway below.

**Pathway 2-8:** The reaction system comprised 100 mM Hepes buffer with pH of 7.5, 100 mM NaCl, 5 mM Mg²⁺, 10 µM Zn²⁺, 1 U/mL AOX, 300 U/mL CAT, 20 mM methanol and 5 mg/mL FLS. The reaction was performed for 2 h, and the yield of formaldehyde was 2.1 mM.

Assay method for compound D: 5 mM dilute sulfuric acid was used as the mobile phase; an HX87 column (Bio-Rad, Aminex@, 300 mm × 78 mm) was used; the flow rate was 0.6 mL/min; the injection amount was 10 µL/injection. The yield of compound D was calculated from the DHA standard curve. the yield of the pathway 2-8 from methanol to compound D was shown in FIG. 4 .

### Example 3. Synthesis of Compound D, Namely Dihydroxyacetone, from Formaldehyde

The synthesis of compound D from formaldehyde can be achieved via the pathway below.

**Pathway** 8: The reaction system comprised 100 mM Hepes buffer with pH of 7.5, 100 mM NaCl, 5 mM Mg²⁺, 10 µM Zn²⁺, 25 mM formaldehyde, 0.5 mM TPP and 10 mg/mL FLS (derived from *Pseudomonas putida*). The reaction was performed for 2 h. Compound D was detected using the method in Example 2, and the result showed that the yield of compound D synthesized using formolase (FLS) was 7.092 mM.

### Example 4. Synthesis of Compound D, Namely Dihydroxyacetone, from Formic Acid

The synthesis of compound D from formic acid can be achieved via the pathway below.

**Pathway 5-6-7-8:** The reaction system comprised 100 mM Hepes buffer with pH of 7.5, 100 mM NaCl, 5 mM Mg²⁺, 10 µM Zn²⁺, 50 mM sodium formate, 0.5 mM TPP, 0.1 mM CoA, 10 mM ATP, 2 mM β-mercaptoethanol, 0.24 mg/mL ACKA, 1.2 mg/mL PTA, 0.2 mg/mL ACDH, 0.024 mg/mL FDH and 0.5 mM NADH. After the reaction was performed at 30 °C for 1-1.5 h, 7.5 mg/mL FLS (derived from *Pseudomonas putida*) was added. After the reaction was performed at 30 °C for another 3.5 h, compound D was detected by gas chromatography-mass spectrometry, and the result showed that the yield of compound D synthesized via the pathway 5-6-7-8 was 0.23 mM.

The gas chromatography-mass spectrometry detection of compound D was performed as follows:
1) Sample preparation: 500 µL of properly diluted sample was reacted with 100 µL of 200 mM PFBOA (O-(2,3,4,5,6-Pentafluorobenzyl) hydroxylamine hydrochloride) at 30 °C for 1 h. 100 µL of n-hexane was added for extraction. 40 µL of the organic phase was added to an equal volume of MSTFA (N-methyl-N-(trimethylsilyl)trifluoroacetamide), and the mixture was reacted at 37 °C for 2 h.
2) Gas chromatography conditions: Agilent 7890A gas chromatograph, helium as carrier gas, carrier gas flow rate of 1.2 mL/min, DB-5MS Ultra Inert capillary column (30 m × 250 µm × 0.25 µm) as chromatography column, initial temperature of 60 °C, maintained for 1 min, running for 1 min, warming rate 1, 5 °C/min to 240 °C, running for 9 min, warming rate 2, 25 °C/min to 300 °C, maintained for 5 min, running for 22 min, injection amount of 1 µL/injection, injector temperature of 250 °C.
3) Mass spectrometry conditions: Agilent 7200 Q-TOF mass spectrometer, solvent delay set to 4 min, EI ionization mode, electron energy of 70 eV, ion source temperature: 230 °C, scan range: 35-550 amu, mass spectrometry acquisition rate of 5 spectra/s.
4) Data analysis: The target metabolites were determined using the Mass Hunter software Qualitative Analysis by making comparisons to the NIST chemical database and by manual analysis, and the yield of DHA was calculated.

### Example 5. Synthesis of Compound F, Namely D-Glyceraldehyde 3-Phosphate, from Compound C, Namely Formaldehyde

The conversion of compound C into compound F can be achieved via Pathway 8-9-10 (the reactions were numbered as shown in FIG. 1). First, the catalysts capable of catalyzing the chemical reactions of the pathway were selected (see Table 1) ; however, enzymes having the catalytic functions are not limited to those listed in Table 1. Then different catalysts were combined according to the pathway, and corresponding reaction system was established. After the reactions were performed for a period of time, the yield of compound F was determined.

Compound E and compound F are isomers, but the cumulative amount of compound F would be low due to the unfavorable free energy, so the yield of compound E + compound F was determined. The yield of compound E + compound F was determined as follows: a 100 µL assay system comprised 100 mM Hepes buffer with pH of 7.5, 100 mM NaCl, 20 U/mL glyceraldehyde phosphate dehydrogenase, 20 U/mL triose phosphate isomerase, 1 mM NAD⁺ and 4 mM potassium arsenate; after the reaction was terminated, a proper dilution of the test sample was assayed for the change in OD340. The yield of compound E + compound F was calculated from the standard curve for glyceraldehyde 3-phosphate.

**Pathway 8-9-10:** The reaction system comprised 100 mM Hepes buffer with pH of 7.5, 100 mM NaCl, 5 mM Mg²⁺, 10 µM Zn²⁺, 10 mM ATP, 0.5 mM thiamine pyrophosphate, 5 mg/mL FLS, 0.1 mg/mL DAK, 0.14 mg/mL TPI and 10 mM compound C. The reaction was performed for 2 h, and the yield of compound E + compound F was 1.25 mM.

### Example 6. Synthesis of Compound I, Namely D-Glucose-6-Phosphate, from Compound F, Namely D-Glyceraldehyde 3-Phosphate

The conversion of compound F into compound I can be achieved via three pathways: Pathway 11-12-15, Pathway 13-15 and Pathway 14-15 (the reactions were numbered as shown in FIG. 1). First, the catalysts capable of catalyzing the chemical reactions of the pathways (see Table 1) were selected; however, enzymes having the catalytic functions are not limited to those listed in Table 1. Then different catalysts were combined according to the pathways, and corresponding reaction systems were established. After the reactions were performed for a period of time, the yield of compound I was determined.

The yield of compound I was determined as follows: a 100 µL assay system comprised 100 mM Hepes buffer with pH of 7.5, 100 mM NaCl, 20 U/mL glucose 6-phosphate dehydrogenase and 1 mM NAD⁺; after 20 µL of the reaction was terminated, a proper dilution of the test sample was assayed for the change in OD340. The yield of compound I was calculated from the standard curve for glucose 6-phosphate.

**Pathway 11-12-15:** The reaction system comprised 100 mM Hepes buffer with pH of 7.5, 100 mM NaCl, 5 mM Mg²⁺, 10 µM Zn²⁺, 3 mM compound F, 3 mM compound E, 0.1 mg/mL FBA, 0.2 mg/mL FBP and 0.17 mg/mL PGI. The reaction was performed for 0.5 h, and the yield of compound I was 2.3 mM.

**Pathway 13-15:** The reaction system comprised 100 mM Hepes buffer with pH of 7.5, 100 mM NaCl, 5 mM Mg²⁺, 10 µM Zn²⁺, 3 mM compound F, 3 mM compound D, 0.3 mg/mL FSA and 0.17 mg/mL PGI. The reaction was performed for 0.5 h, and the yield of compound I was 1.28 mM.

**Pathway 14-15:** The reaction system comprised 100 mM Hepes buffer with pH of 7.5, 100 mM NaCl, 5 mM Mg²⁺, 10 µM Zn²⁺, 3 mM compound F, 3 mM compound E, 0.3 mg/mL FBAP and 0.17 mg/mL PGI. The reaction was performed for 0.5 h, and the yield of compound I was 0.13 mM.

### Example 7. Synthesis of Compound 1, Namely Amylose, from Compound I, Namely D-Glucose-6-Phosphate

The conversion of compound I into compound 1 can be achieved via two pathways: Pathway 16-17-18 and Pathway 16-19 (the reactions were numbered as shown in FIG. 1). First, the catalysts capable of catalyzing the chemical reactions of the pathways (see Table 1) were selected; however, enzymes having the catalytic functions are not limited to those listed in Table 1. Then different catalysts were combined according to the pathways, and corresponding reaction systems were established. After the reactions were performed for a period of time, the yield of compound 1 was determined.

The yield of compound 1 was determined as follows: after the reaction was terminated, the sample was properly diluted, and then incubated with 30 U/mL α-amylase and 33 U/mL glucoamylase for a certain period of time until compound 1 was completely hydrolyzed to glucose, and then the glucose content was determined using a glucose assay kit (Beijing Applygen Technologies Inc., E1010). The yield of compound 1 is expressed in terms of the glucose content.

**Pathway 16-17-18:** The reaction system comprised 100 mM Hepes buffer with pH of 7.5, 100 mM NaCl, 5 mM Mg²⁺, 10 µM Zn²⁺, 5 mM compound I, 10 mM ATP, 10 mg/L dextrin, 0.275 mg/mL PGM, 0.46 mg/mL GlgC, 0.2 mg/mL PPase and 0.235 mg/mL GlgA. The reaction was performed for 3 h, and the yield of compound 1 was 436.8 mg/L.

**Pathway 16-19:** The reaction system comprised 100 mM Hepes buffer with pH of 7.5, 100 mM NaCl, 5 mM Mg²⁺, 10 µM Zn²⁺, 5 mM compound I, 10 mg/L dextrin, 0.275 mg/mL PGM and 0.13 mg/mL αGP. The reaction was performed for 3 h, and the yield of compound 1 was 100.7 mg/L.

### Example 8. Synthesis of Compound 2, Namely Amylopectin, from Compound 1, Namely Amylose

The conversion of compound 1 into compound 2 can be achieved via Pathway 20 (the reactions were numbered as shown in FIG. 1). First, the catalyst capable of catalyzing the chemical reaction 20 (see Table 1) was selected; however, an enzyme having the catalytic function is not limited to that listed in Table 1. Then different catalysts were combined according to the pathways, and corresponding reaction systems were established. After the reactions were performed for a period of time, the yield of compound 2 was determined.

The yield of compound 2 was determined as follows: 1) qualitative detection of compound 2: based on the principle that amylose turns blue in the presence of iodine solution (the maximum absorption wavelength is about 620 nm), and that amylopectin turns purple in the presence of iodine solution (the maximum absorption wavelength is about 530 nm), whether compound 2 was produced was qualitatively determined by detecting the change in the maximum absorption wavelength of the compound before and after reaction by iodine staining; 2) quantitative measurement of compound 2: after the reaction was terminated, a proper dilution of the sample was incubated with 30 U/mL α-amylase and 33 U/mL glucoamylase for a certain period of time until compound 2 was completely hydrolyzed to glucose, and then the glucose content was determined using a glucose assay kit (Beijing Applygen Technologies Inc., E1010). The yield of compound 2 is expressed in terms of the glucose content.

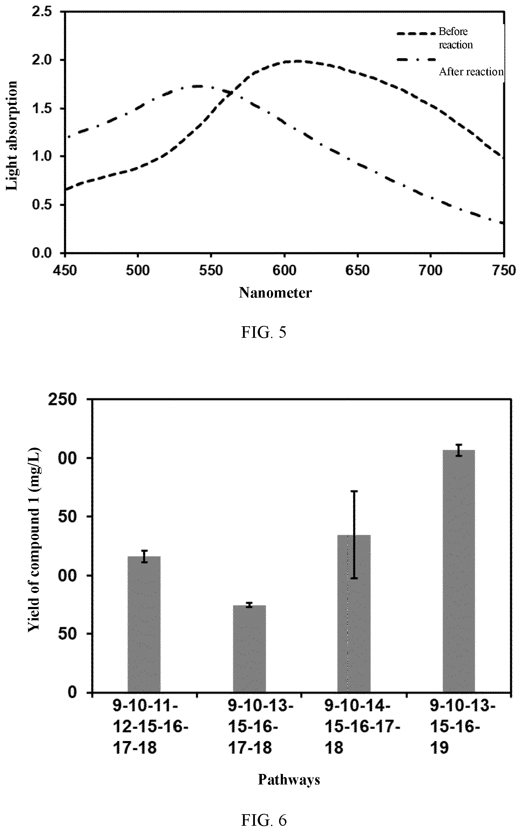

**Pathway 20:** The reaction system comprised 100 mM Hepes buffer with pH of 7.5, 100 mM NaCl, 5 mM Mg²⁺, 10 µM Zn²⁺, 3 g/L compound 1 and 0.05 mg/mL GlgB. The reaction was performed for 3 h, and the yield of compound 2 was 2.7 g/L. FIG. 5 shows full-wavelength scanning spectra before and after the reaction of the pathway from compound 1 to compound 2.

### Example 9. Synthesis of Compound 1, Namely Amylose, from Compound D, Namely Dihydroxyacetone

The conversion of compound D into compound I can be achieved via the pathway below.

**Pathway 9-10-11-12-15-16-17-18:** The reaction system comprised 100 mM Hepes buffer with pH of 7.5, 100 mM NaCl, 5 mM Mg²⁺, 10 µM Zn²⁺, 0.077 mg/mL DAK, 0.33 mg/mL TPI, 0.15 mg/mL FBA, 0.6 mg/mL FBP-M, 0.069 mg/mL PGI, 0.565 mg/mL PGM, 0.5 mg/mL GlgA, 1 mg/mL GlgC-M, 0.1 mM EDTA, 1 mM ADP, 0.2 mM polyphosphoric acid, 0.22 mg/mL PPK (polyphosphate kinase, for regenerating ATP), 3 mM compound D and 10 mg/L dextrin. The reaction was performed for 5 h, and the yield of compound 1 was 116.2 mg/L.

**Pathway 9-10-13-15-16-17-18:** The reaction system comprised 100 mM Hepes buffer with pH of 7.5, 100 mM NaCl, 5 mM Mg²⁺, 10 µM Zn²⁺, 0.077 mg/mL DAK, 0.33 mg/mL TPI, 0.3 mg/mL FSA, 0.069 mg/mL PGI, 0.565 mg/mL PGM, 0.5 mg/mL GlgA, 1 mg/mL GlgC-M, 0.1 mM EDTA, 1 mM ADP, 0.2 mM polyphosphoric acid, 0.22 mg/mL PPK (polyphosphate kinase, for regenerating ATP), 3 mM compound D and 10 mg/L dextrin. The reaction was performed for 5 h, and the yield of compound 1 was 74.5 mg/L.

**Pathway 9-10-14-15-16-17-18:** The reaction system comprised 100 mM Hepes buffer with pH of 7.5, 100 mM NaCl, 5 mM Mg²⁺, 10 µM Zn²⁺, 0.077 mg/mL DAK, 0.33 mg/mL TPI, 2.5 mg/mL FBAP, 0.069 mg/mL PGI, 0.565 mg/mL PGM, 0.5 mg/mL GlgA, 1 mg/mL GlgC-M, 0.1 mM EDTA, 1 mM ADP, 0.2 mM polyphosphoric acid, 0.22 mg/mL PPK (polyphosphate kinase, for regenerating ATP), 3 mM compound D and 10 mg/L dextrin. The reaction was performed for 5 h, and the yield of compound 1 was 134.4 mg/L.

**Pathway 9-10-13-15-16-19:** The reaction system comprised 100 mM Hepes buffer with pH of 7.5, 100 mM NaCl, 5 mM Mg²⁺, 10 µM Zn²⁺, 0.077 mg/mL DAK, 0.33 mg/mL TPI, 0.3 mg/mL FSA, 0.069 mg/mL PGI, 0.565 mg/mL PGM, 1 mg/mL αGP, 0.1 mM EDTA, 1 mM ADP, 0.2 mM polyphosphoric acid, 0.22 mg/mL PPK (polyphosphate kinase, for regenerating ATP), 10 mM compound D and 10 mg/L dextrin. The reaction was performed for 23 h, and the yield of compound 1 was 206.55 mg/L. FIG. 6 shows the yields of the pathways from compound D to compound 1.
FBP-M used in Example 9 is a mutant of fructose-bisphosphatase (FBP), which comprises a total of four mutated sites: lysine (encoded by AAA) at position 104 mutated to glutamine (encoded by CAG), arginine (encoded by CGC) at position 132 mutated to isoleucine (encoded by ATT), tyrosine (encoded by TAC) at position 210 mutated to phenylalanine (encoded by TTT), and lysine (encoded by AAG) at position 218 mutated to glutamine (encoded by CAG). GlgC-M is a mutant of glucose-1-phosphate adenylyltransferase (GlgC), which comprises a total of two mutated sites: proline (encoded by CCG) at position 295 mutated to aspartic acid (encoded by GAT), and glycine (encoded by GGC) at position 336 mutated to aspartic acid (encoded by GAT). Gene fragments comprising the desired mutations were obtained by fusion PCR and were all cloned into pET21b vector (Novagen, Madison, WI) by simple cloning (You, C., et al. (2012). "Simple Cloning via Direct Transformation of PCR Product (DNA Multimer) to Escherichia coli and Bacillus subtilis." Appl. Environ. Microbiol. 78(5):1593-1595.) to obtain corresponding expression vectors pET21b-FBP-M and pET21b-GlgC-M. These two plasmids were transformed into *Escherichia coli* expression system BL21(DE3) (Invitrogen, Carlsbad, CA), and the proteins were expressed and purified.

### Example 10. Synthesis of Compound 2, Namely Amylopectin, from Compound D, Namely Dihydroxyacetone

The synthesis of compound 2 from compound D can be achieved via the pathway below.

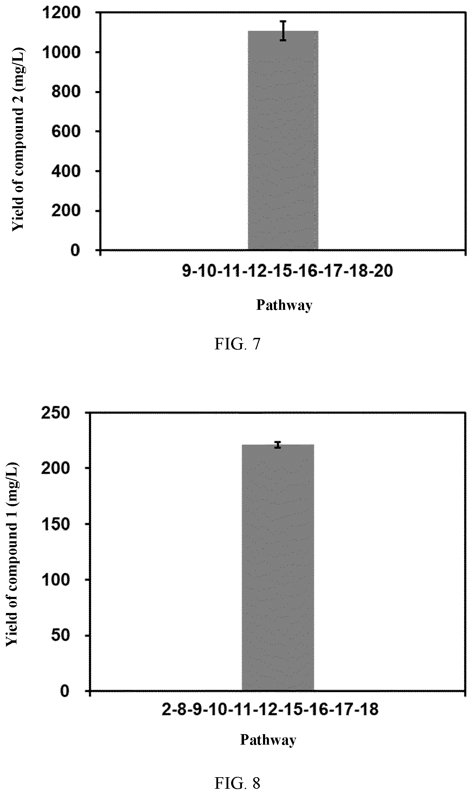

**Pathway 9-10-11-12-15-16-17-18-20:** The reaction system comprised 100 mM Hepes buffer with pH of 7.5, 100 mM NaCl, 5 mM Mg²⁺, 10 µM Zn²⁺, 0.77 mg/mL DAK, 0.33 mg/mL TPI, 0.15 mg/mL FBA, 0.43 mg/mL FBP-M, 0.1 mg/mL PGI, 0.565 mg/mL PGM, 0.47 mg/mL GlgA, 0.92 mg/mL GlgC-M, 0.02 mg/mL GlgB, 0.1 mM EDTA, 1 mM ADP, 0.4 mM polyphosphoric acid (additional 0.2 mM was added per hour), 0.44 mg/mL PPK (polyphosphate kinase, for regenerating ATP), 20 mM compound D and 0.1 g/L dextrin. The reaction was performed for 4 h, and the yield of compound 2 was 1107.77 mg/L. FIG. 7 shows the yield of the pathway from compound D to compound 2.

### Example 11. Synthesis of Compound 1, Namely Amylose, from Methanol

The synthesis of compound 1 from methanol can be achieved via the pathway below.

**Pathway 2-8-9-10-11-12-15-16-17-18:** The reaction system comprised 100 mM Hepes buffer with pH of 7.5, 100 mM NaCl, 5 mM Mg²⁺, 10 µM Zn²⁺, 1 U/mL AOX, 300 U/mL CAT, 5 mg/mL FLS-M, 0.5 mM thiamine pyrophosphate, 0.035 mg/mL DAK, 0.33 mg/mL TPI, 0.05 mg/mL FBA, 0.2 mg/mL FBP-M, 0.023 mg/mL PGI, 0.11 mg/mL PGM, 0.1 mg/mL GlgA, 0.2 mg/mL GlgC-M, 0.1 mM EDTA, 1 mM ADP, 0.2 mM polyphosphoric acid, 0.22 mg/mL PPK (polyphosphate kinase, for regenerating ATP), 20 mM methanol and 0.01 g/L dextrin. The reaction was performed for 6 h, and the yield of compound 1 was 221.1 mg/L. FIG. 8 shows the yield of the synthesis pathway from methanol to compound 1.

FLS-M used in Example 11 is a mutant of formolase (FLS), which comprises a total of three mutated sites: isoleucine (encoded by ATT) at position 28 mutated to leucine (encoded by CTA), threonine (encoded by ACC) at position 90 mutated to leucine (encoded by CTG), and asparagine (encoded by AAC) at position 283 mutated to histidine (encoded by CAT). Gene fragments comprising the desired mutations were obtained by fusion PCR and were cloned into pET21a vector (Novagen, Madison, WI) by simple cloning (You, C., et al. (2012). "Simple Cloning via Direct Transformation of PCR Product (DNA Multimer) to Escherichia coli and Bacillus subtilis." Appl. Environ. Microbiol. 78(5):1593-1595.) to obtain corresponding expression vector pET21a-FLS-M. These two plasmids were transformed into *Escherichia coli* expression system BL21(DE3) (Invitrogen, Carlsbad, CA), and the proteins were expressed and purified.
FBP-M used in Example 11 was the same as that used in Example 9.

The above examples show that the total artificial biosynthesis of starch from simple compounds such as dihydroxyacetone, formaldehyde, formic acid and methanol and the like can be achieved by using the methods of the present disclosure and with short periods and high yields.

The embodiments of the present disclosure have been described above. However, the present disclosure is not limited to the embodiments described above. Any modification, equivalent, improvement and the like made without departing from the spirit and principle of the present disclosure shall fall within the protection scope of the present disclosure.

## Claims

1. A method (I) for synthesis of starch, comprising pathway of converting starting material compound D, namely dihydroxyacetone, into starch by catalysis with multiple enzymes;
wherein the pathway comprises the following steps:
step (1): converting the starting material compound D, namely dihydroxyacetone, into a compound F, namely D-glyceraldehyde 3-phosphate, by catalysis with one or more enzymes;
step (2): converting the compound F obtained in step (1) into a compound I, namely D-glucose-6-phosphate, by catalysis with one or more enzymes; and
step (3): converting the compound I obtained in step (2) into starch by catalysis with one or more enzymes;
preferably, the enzyme used in step (1) is an enzyme or a combination of enzymes which catalyzes conversion of dihydroxyacetone into D-glyceraldehyde 3-phosphate by one-step or multi-step reaction; for example, the enzyme may be a combination of an enzyme having the function of catalyzing conversion of dihydroxyacetone into dihydroxyacetone phosphate and an enzyme having the function of catalyzing conversion of dihydroxyacetone phosphate into D-glyceraldehyde 3-phosphate;
the enzyme used in step (2) is an enzyme or a combination of enzymes which catalyzes conversion of D-glyceraldehyde 3-phosphate into D-glucose-6-phosphate by one-step or multi-step reaction; for example, the enzyme may be the following enzyme combinations: an enzyme combination (I-2-a): a combination of an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate and an enzyme having the function of catalyzing conversion of D-fructose-6-phosphate into D-glucose-6-phosphate; or an enzyme combination (I-2-b): a combination of an enzyme having the function of catalyzing conversion of D-glyceraldehyde 3-phosphate into D-fructose-6-phosphate and an enzyme having the function of catalyzing conversion of D-fructose-6-phosphate into D-glucose-6-phosphate;
the enzyme used in step (3) is an enzyme or a combination of enzymes which catalyzes conversion of D-glucose-6-phosphate into amylose or amylopectin by one-step or multi-step reaction; for example, the enzyme may be the following enzyme combinations: an enzyme combination (I-3-a): a combination of an enzyme having the function of catalyzing conversion of D-glucose-6-phosphate into α-D-glucose-1-phosphate and an enzyme having the function of catalyzing conversion of α-D-glucose-1-phosphate into amylose; or an enzyme combination (I-3-b): a combination of an enzyme having the function of catalyzing conversion of D-glucose-6-phosphate into α-D-glucose-1-phosphate, an enzyme having the function of catalyzing conversion of α-D-glucose-1-phosphate into adenosine diphosphate-α-D-glucose and an enzyme having the function of catalyzing conversion of adenosine diphosphate-α-D-glucose into amylose; optionally, the combination (I-3-a) or (I-3-b) may also comprise an enzyme having the function of catalyzing conversion of amylose into amylopectin.

2. The method (I) for synthesis of starch as claimed in claim 1, wherein step (1) comprises the following sub-steps:
step (1-1): converting the compound D into a compound E, namely dihydroxyacetone phosphate, by catalysis with one or more enzymes (the reaction is denoted by reaction 9); and
step (1-2): converting the compound E obtained in step (1-1) into the compound F, namely D-glyceraldehyde 3-phosphate, by catalysis with one or more enzymes (the reaction is denoted by reaction 10);
preferably, the enzyme used in step (1-1) is an enzyme having the function of catalyzing conversion of dihydroxyacetone into dihydroxyacetone phosphate; the enzyme used in step (1-2) is an enzyme having the function of catalyzing conversion of dihydroxyacetone phosphate into D-glyceraldehyde 3-phosphate;
step (2) comprises the following sub-steps:
step (2-1): converting the compound F into a compound H, namely D-fructose-6-phosphate, by catalysis with one or more enzymes; and
step (2-2): converting the compound H obtained in step (2-1) into the compound I, namely D-glucose-6-phosphate, by catalysis with one or more enzymes (the reaction is denoted by reaction 15);
preferably, the enzyme used in step (2-1) is an enzyme or a combination of enzymes which catalyzes conversion of D-glyceraldehyde 3-phosphate into D-fructose-6-phosphate by one-step or multi-step reaction; for example, the enzyme may be a single enzyme having the function of catalyzing conversion of D-glyceraldehyde 3-phosphate into D-fructose-6-phosphate, or may be an enzyme combination (I-2-1): a combination of an enzyme having the function of catalyzing conversion of D-glyceraldehyde 3-phosphate into D-fructose-1,6-bisphosphate and an enzyme having the function of catalyzing conversion of D-fructose-1,6-bisphosphate into D-fructose-6-phosphate; the enzyme used in step (2-2) is an enzyme having the function of catalyzing conversion of D-fructose-6-phosphate into D-glucose-6-phosphate;
more preferably, step (2-1) may be done by: converting the compound F into the compound H by catalysis with an enzyme having the function of catalyzing conversion of D-glyceraldehyde 3-phosphate into D-fructose-6-phosphate (the reaction is denoted by reaction 13 or 14); or step (2-1) may also be done by: converting first the compound F into a compound G, namely D-fructose-1,6-bisphosphate, by catalysis with an enzyme having the function of catalyzing conversion of D-glyceraldehyde 3-phosphate into D-fructose-1,6-bisphosphate (the reaction is denoted by reaction 11), and then converting the obtained compound G into D-fructose-6-phosphate by catalysis with an enzyme having the function of catalyzing conversion of D-fructose-1,6-bisphosphate into D-fructose-6-phosphate (the reaction is denoted by reaction 12);
step (3) comprises the following sub-steps:
step (3-1): converting the compound I into a compound J, namely α-D-glucose-1-phosphate, by catalysis with one or more enzymes (the reaction is denoted by reaction 16);
step (3-2): converting the compound J obtained in step (3-1) into a compound 1, namely amylose, by catalysis with one or more enzymes; and
optional step (3-3): converting the compound 1 obtained in step (3-2) into a compound 2, namely amylopectin, by catalysis with one or more enzymes (the reaction is denoted by reaction 20);
preferably, the enzyme used in step (3-1) is an enzyme having the function of catalyzing conversion of D-glucose-6-phosphate into α-D-glucose-1-phosphate;
the enzyme used in step (3-2) is an enzyme having the function of catalyzing conversion of α-D-glucose-1-phosphate into amylose; for example, the enzyme may be a single enzyme having the function of catalyzing conversion of α-D-glucose-1-phosphate into amylose, or may be an enzyme combination (I-3-2): a combination of an enzyme having the function of catalyzing conversion of α-D-glucose-1-phosphate into adenosine diphosphate-α-D-glucose and an enzyme having the function of catalyzing conversion of adenosine diphosphate-α-D-glucose into amylose;
the enzyme used in step (3-3) is an enzyme having the function of catalyzing conversion of amylose into amylopectin;
more preferably, step (3-2) may be done by: converting the compound J into the compound 1, namely amylose, by catalysis with an enzyme having the function of catalyzing conversion of α-D-glucose-1-phosphate into amylose (the reaction is denoted by reaction 19); or step (3-2) may also be done by: first, converting the compound J into a compound K, namely adenosine diphosphate-α-D-glucose, by catalysis with an enzyme having the function of catalyzing conversion of α-D-glucose-1-phosphate into adenosine diphosphate-α-D-glucose (the reaction is denoted by reaction 17), and then converting the obtained compound K into amylose by catalysis with an enzyme having the function of catalyzing adenosine diphosphate-α-D-glucose into amylose (the reaction is denoted by reaction 18).

3. The method (I) for synthesis of starch as claimed in claim 1 or 2, wherein the method (I) further comprises, before step (1), step (0) of converting starting material formaldehyde into the compound D, namely dihydroxyacetone, by catalysis with one or more enzymes, and the reaction is denoted by reaction 8; more preferably, the enzyme used in step (0) is an enzyme having the function of catalyzing conversion of formaldehyde into dihydroxyacetone.

4. The method (I) for synthesis of starch as claimed in claim 3, wherein the method (I) further comprises, before step (0), step (a) of converting starting material methanol or formic acid into formaldehyde by catalysis with one or more enzymes; more preferably, in step (a), when methanol is used as a starting material to synthesize formaldehyde, the enzyme used is an enzyme having the function of catalyzing conversion of methanol into formaldehyde; when formic acid is used as a starting material to synthesize formaldehyde, the enzyme used is an enzyme having the function of catalyzing conversion of formic acid into formaldehyde; for example, the enzyme may be a single enzyme having the function of catalyzing conversion of formic acid into formaldehyde, or may be an enzyme combination (I-a-1): a combination of an enzyme having the function of catalyzing conversion of formic acid into formyl coenzyme A and an enzyme having the function of catalyzing conversion of formyl coenzyme A into formaldehyde, or an enzyme combination (I-a-2): a combination of an enzyme having the function of catalyzing conversion of formic acid into formyl phosphate, an enzyme having the function of catalyzing conversion of formyl phosphate into formyl coenzyme A, and an enzyme having the function of catalyzing conversion of formyl coenzyme A into formaldehyde;
more preferably, in step (a), when formic acid is used as a starting material to synthesize formaldehyde, any one of the following steps (a1), (a2) and (a3) can be followed:
step (a1): converting starting material formic acid into formaldehyde by catalysis with an enzyme having the function of catalyzing conversion of formic acid into formaldehyde (the reaction is denoted by reaction 3);
step (a2): first, converting starting material formic acid into formyl coenzyme A by catalysis with an enzyme having the function of catalyzing conversion of formic acid into formyl coenzyme A (the reaction is denoted by reaction 4), and then converting formyl coenzyme A into formaldehyde by catalysis with an enzyme having the function of catalyzing conversion of formyl coenzyme A into formaldehyde (the reaction is denoted by reaction 7); or
step (a3): first, converting starting material formic acid into formyl phosphate by catalysis with an enzyme having the function of catalyzing conversion of formic acid into formyl phosphate (the reaction is denoted by reaction 5), then converting formyl phosphate into formyl coenzyme A by catalysis with an enzyme having the function of catalyzing conversion of formyl phosphate into formyl coenzyme A (the reaction is denoted by reaction 6), and then converting formyl coenzyme A into formaldehyde by catalysis with an enzyme having the function of catalyzing conversion of formyl coenzyme A into formaldehyde (the reaction is denoted by reaction 7).

5. A method (II) for the synthesis of starch, comprising the following steps:
step 1): converting starting material methanol into amylose by catalysis with multiple enzymes; and optional step 2): converting starting material amylose into amylopectin by catalysis with one or more enzymes;
preferably, the enzyme used in step 1) is a combination of enzymes which catalyzes synthesis of starch from methanol by multi-step reaction; for example, the enzyme may be the following enzyme combinations:
an enzyme combination (II-1-a): a combination of an enzyme having the function of catalyzing conversion of methanol into formaldehyde, an enzyme having the function of catalyzing conversion of formaldehyde into dihydroxyacetone, an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate, an enzyme having the function of catalyzing conversion of α-D-glucose-1-phosphate into adenosine diphosphate-α-D-glucose and an enzyme having the function of catalyzing conversion of adenosine diphosphate-α-D-glucose into amylose;
an enzyme combination (II-1-b): a combination of an enzyme having the function of catalyzing conversion of methanol into formaldehyde, an enzyme having the function of catalyzing conversion of formaldehyde into dihydroxyacetone, an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate, an enzyme having the function of catalyzing conversion of α-D-glucose-1-phosphate into adenosine diphosphate-α-D-glucose and an enzyme having the function of catalyzing conversion of adenosine diphosphate-α-D-glucose into amylose;
an enzyme combination (II-1-c): a combination of an enzyme having the function of catalyzing conversion of methanol into formaldehyde, an enzyme having the function of catalyzing conversion of formaldehyde into dihydroxyacetone, an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate and an enzyme having the function of catalyzing conversion of α-D-glucose-1-phosphate into amylose; or
an enzyme combination (II-1-d): a combination of an enzyme having the function of catalyzing conversion of methanol into formaldehyde, an enzyme having the function of catalyzing conversion of formaldehyde into dihydroxyacetone, an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate and an enzyme having the function of catalyzing conversion of α-D-glucose-1-phosphate into amylose;
the enzyme used in step 2) is an enzyme having the function of catalyzing conversion of amylose into amylopectin.

6. A method (III) for the synthesis of starch, comprising the following steps:
step 1): converting starting material methanol into a compound D, namely dihydroxyacetone, by catalysis with one or more enzymes;
step 2): converting the dihydroxyacetone obtained in step 1) into amylose by catalysis with one or more enzymes; and
optional step 3): converting the amylose obtained in step 2) into amylopectin by catalysis with one or more enzymes;
preferably, the enzyme used in step 1) is an enzyme combination (III-1): a combination of an enzyme having the function of catalyzing conversion of methanol into formaldehyde and an enzyme having the function of catalyzing conversion of formaldehyde into dihydroxyacetone;
the enzyme used in step 2) is the following enzyme combinations:
an enzyme combination (III-2-a): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate, an enzyme having the function of catalyzing conversion of α-D-glucose-1-phosphate into adenosine diphosphate-α-D-glucose and an enzyme having the function of catalyzing conversion of adenosine diphosphate-α-D-glucose into amylose;
an enzyme combination (III-2-b): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate, an enzyme having the function of catalyzing conversion of α-D-glucose-1-phosphate into adenosine diphosphate-α-D-glucose and an enzyme having the function of catalyzing conversion of adenosine diphosphate-α-D-glucose into amylose;
an enzyme combination (III-2-c): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate and an enzyme having the function of catalyzing conversion of α-D-glucose-1-phosphate into amylose; or
an enzyme combination (III-2-d): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate and an enzyme having the function of catalyzing conversion of α-D-glucose-1-phosphate into amylose;
the enzyme used in step 3) is an enzyme having the function of catalyzing conversion of amylose into amylopectin.

7. A method (IV) for the synthesis of starch, comprising the following steps:
step 1): converting starting material formic acid into dihydroxyacetone by catalysis with one or more enzymes;
step 2): converting the dihydroxyacetone obtained in step 1) into amylose by catalysis with one or more enzymes; and
optional step 3): converting the amylose obtained in step 2) into amylopectin by catalysis with one or more enzymes;
preferably, the enzyme used in step 1) is an enzyme or a combination of enzymes which catalyzes conversion of formic acid into dihydroxyacetone by one-step or multi-step reaction; for example, the enzyme may be the following enzyme combinations:
an enzyme combination (IV-1-a): a combination of an enzyme having the function of catalyzing conversion of formic acid into formaldehyde and an enzyme having the function of catalyzing conversion of formaldehyde into dihydroxyacetone;
an enzyme combination (IV-1-b): a combination of an enzyme having the function of catalyzing conversion of formic acid into formyl coenzyme A, an enzyme having the function of catalyzing conversion of formyl coenzyme A into formaldehyde and an enzyme having the function of catalyzing conversion of formaldehyde into dihydroxyacetone; or
an enzyme combination (IV-1-c): a combination of an enzyme having the function of catalyzing conversion of formaldehyde into dihydroxyacetone, an enzyme having the function of catalyzing conversion of dihydroxyacetone into dihydroxyacetone phosphate and an enzyme having the function of catalyzing conversion of dihydroxyacetone phosphate into D-glyceraldehyde 3-phosphate;
the enzyme used in step 2) is an enzyme or a combination of enzymes which catalyzes conversion of dihydroxyacetone into amylose by one-step or multi-step reaction; for example, the enzyme may be following enzyme combinations:
an enzyme combination (IV-2-a): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate and an enzyme having the function of catalyzing conversion of α-D-glucose-1-phosphate into amylose;
an enzyme combination (IV-2-b): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate, an enzyme having the function of catalyzing conversion of α-D-glucose-1-phosphate into adenosine diphosphate-α-D-glucose and an enzyme having the function of catalyzing conversion of adenosine diphosphate-α-D-glucose into amylose;
an enzyme combination (IV-2-c): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate and an enzyme having the function of catalyzing conversion of α-D-glucose-1-phosphate into amylose; or
an enzyme combination (IV-2-d): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate, an enzyme having the function of catalyzing conversion of α-D-glucose-1-phosphate into adenosine diphosphate-α-D-glucose and an enzyme having the function of catalyzing conversion of adenosine diphosphate-α-D-glucose into amylose;
the enzyme used in step 3) is an enzyme having the function of catalyzing conversion of amylose into amylopectin.

8. A method (V) for the synthesis of starch, comprising the following steps:
step 1): converting starting material formic acid into formaldehyde by catalysis with one or more enzymes;
step 2): converting the formaldehyde obtained in step 1) into dihydroxyacetone by catalysis with one or more enzymes;
step 3): converting the dihydroxyacetone obtained in step 2) into amylose by catalysis with one or more enzymes; and
optional step 4): converting the amylose obtained in step 3) into amylopectin by catalysis with one or more enzymes;
preferably, the enzyme used in step 1) is a single enzyme having the function of catalyzing conversion of formic acid into formaldehyde, or the following enzyme combinations: an enzyme combination (V-1-a): an enzyme having the function of catalyzing conversion of formic acid into formyl coenzyme A and an enzyme having the function of catalyzing conversion of formyl coenzyme A into formaldehyde, or an enzyme combination (V-1-b): a combination of an enzyme having the function of catalyzing conversion of formic acid into formyl phosphate, an enzyme having the function of catalyzing conversion of formyl phosphate into formyl coenzyme A and an enzyme having the function of catalyzing conversion of formyl coenzyme A into formaldehyde;
the enzyme used in step 2) is an enzyme having the function of catalyzing conversion of formaldehyde into dihydroxyacetone;
the enzyme used in step 3) is the following enzyme combinations:
an enzyme combination (V-3-a): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate and an enzyme having the function of catalyzing conversion of α-D-glucose-1-phosphate into amylose;
an enzyme combination (V-3-b): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate, an enzyme having the function of catalyzing conversion of α-D-glucose-1-phosphate into adenosine diphosphate-α-D-glucose and an enzyme having the function of catalyzing conversion of adenosine diphosphate-α-D-glucose into amylose;
an enzyme combination (V-3-c): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate and an enzyme having the function of catalyzing conversion of α-D-glucose-1-phosphate into amylose; or
an enzyme combination (V-3-d): a combination of an enzyme having the function of catalyzing conversion of dihydroxyacetone into dihydroxyacetone phosphate, an enzyme having the function of catalyzing conversion of dihydroxyacetone phosphate into D-glyceraldehyde 3-phosphate, an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate, an enzyme having the function of catalyzing conversion of D-fructose-6-phosphate into D-glucose-6-phosphate, an enzyme having the function of catalyzing conversion of D-glucose-6-phosphate into α-D-glucose-1-phosphate, an enzyme having the function of catalyzing conversion of α-D-glucose-1-phosphate into adenosine diphosphate-α-D-glucose and an enzyme having the function of catalyzing conversion of adenosine diphosphate-α-D-glucose into amylose;
the enzyme used in step 4) is an enzyme having the function of catalyzing conversion of amylose into amylopectin.

9. A method (VI) for the synthesis of starch, comprising the following steps:
step 1): converting starting material formic acid into formaldehyde by catalysis with one or more enzymes;
step 2): converting the formaldehyde obtained in step 1) into a compound F, namely D-glyceraldehyde 3-phosphate, by catalysis with one or more enzymes;
step 3): converting the D-glyceraldehyde 3-phosphate obtained in step 2) into D-glucose-6-phosphate by catalysis with one or more enzymes;
step 4): converting the D-glucose-6-phosphate obtained in step 3) into amylose by catalysis with one or more enzymes; and
optional step 5): converting the amylose obtained in step 4) into amylopectin by catalysis with one or more enzymes;
preferably, the enzyme used in step 1) is an enzyme combination (VI-1): a combination of an enzyme having the function of catalyzing conversion of formic acid into formyl phosphate, an enzyme having the function of catalyzing conversion of formyl phosphate into formyl coenzyme A and an enzyme having the function of catalyzing conversion of formyl coenzyme A into formaldehyde;
the enzyme used in step 2) is an enzyme combination (VI-2): a combination of an enzyme having the function of catalyzing conversion of formaldehyde into dihydroxyacetone, an enzyme having the function of catalyzing conversion of dihydroxyacetone into dihydroxyacetone phosphate and an enzyme having the function of catalyzing conversion of dihydroxyacetone phosphate into D-glyceraldehyde 3-phosphate;
the enzyme used in step 3) is the following enzyme combinations:
an enzyme combination (VI-3-a): a combination of an enzyme having the function of catalyzing conversion of D-glyceraldehyde 3-phosphate into D-fructose-1,6-bisphosphate, an enzyme having the function of catalyzing conversion of D-fructose-1,6-bisphosphate into D-fructose-6-phosphate and an enzyme having the function of catalyzing conversion of D-fructose-6-phosphate into D-glucose-6-phosphate; or
an enzyme combination (VI-3-b): a combination of an enzyme having the function of catalyzing conversion of D-glyceraldehyde 3-phosphate into D-fructose-6-phosphate and an enzyme having the function of catalyzing conversion of D-fructose-6-phosphate into D-glucose-6-phosphate;
the enzyme used in step 4) is the following enzyme combinations:
an enzyme combination (VI-4-a): a combination of an enzyme having the function of catalyzing conversion of D-glucose-6-phosphate into α-D-glucose-1-phosphate, and enzyme having the function of catalyzing conversion of α-D-glucose-1-phosphate into adenosine diphosphate-α-D-glucose and an enzyme having the function of catalyzing conversion of adenosine diphosphate-α-D-glucose into amylose; or
an enzyme combination (VI-4-b): a combination of an enzyme having the function of catalyzing conversion of D-glucose-6-phosphate into α-D-glucose-1-phosphate and an enzyme having the function of catalyzing conversion of α-D-glucose-1-phosphate into amylose;
the enzyme used in step 5) is an enzyme having the function of catalyzing conversion of amylose into amylopectin.

10. The method for synthesis of starch as claimed in any one of claims 1-9, wherein the steps, sub-steps or specific reactions of the method can be performed step by step, or any adjacent two, three, four, five, six, seven or more steps, sub-steps or specific reactions can also be performed simultaneously, or all the steps or specific reactions can be performed simultaneously.

11. A method for the synthesis of dihydroxyacetone, comprising pathway of converting starting material methanol into dihydroxyacetone by catalysis with one or more enzymes;
wherein the pathway comprises the following steps:
step (1): converting starting material methanol into formaldehyde by catalysis with an enzyme having the function of catalyzing conversion of methanol into formaldehyde; and
step (2): converting the formaldehyde obtained in step (1) into dihydroxyacetone by catalysis with an enzyme having the function of catalyzing conversion of formaldehyde into dihydroxyacetone;
preferably, the enzyme having the function of catalyzing conversion of methanol into formaldehyde in step (1) includes, but is not limited to, alcohol oxidase (AOX) or mutants thereof, cholesterol oxidase or mutants thereof, alcohol dehydrogenase (ADH) or mutants thereof, methanol dehydrogenase or mutants thereof, L-threonine-3-dehydrogenase or mutants thereof, cyclohexanol dehydrogenase or mutants thereof, or *n*-butanol dehydrogenase or mutants thereof;
the enzyme having the function of catalyzing conversion of formaldehyde into dihydroxyacetone in step (2) includes, but is not limited to, formolase (FLS) or mutants thereof (FLS-M), or glycolaldehyde synthase (GALS) or mutants thereof;
wherein steps (1) and (2) can be performed simultaneously or step by step; when steps (1) and (2) are simultaneously performed, a reaction system comprises substrate methanol, the enzyme having the function of catalyzing conversion of methanol into formaldehyde and the enzyme having the function of catalyzing conversion of formaldehyde into dihydroxyacetone; optionally, an auxiliary enzyme such as catalase may also be comprised.
